# EUROPEAN PATENT SPECIFICATION

(11) **EP 3 989 907 B1**
(45) Date of publication and mention of the grant of the patent: **25.10.2023**
(21) Application number: 20746701.0
(22) Date of filing: 26.06.2020
(51) Int. Cl.: A61H 23/02

(54) **WEARABLE DEVICE**
TRAGBARE VORRICHTUNG
DISPOSITIF PORTABLE

(30) Priority: 26.06.2019 GB 201909176
(43) Date of publication of application: 04.05.2022
(73) Proprietor: Charco Neurotech Ltd, London E9 5EN (GB)
(72) Inventor: JUNG, Soo Min, Cambridge CB21 5HW (GB)
(74) Representative: Basck Limited
(86) International application number: PCT/IB2020/056091
(87) International publication number: WO 2020/261225

(56) References cited:
- WO-A1-2013/063420
- WO-A1-2016/051414
- GB-A- 2 539 264
- US-A1- 2012 259 255
- US-A1- 2013 204 169
- US-A1- 2016 058 658
- US-A1- 2018 271 744

## Description

### FIELD OF THE INVENTION

The present invention relates to wearable device and in particular to a wearable device configured to provide a mechanical stimulus.

### BACKGROUND

Individuals who suffer from neurological conditions such as Parkinson's disease or multiple sclerosis (MS), or have suffered a stroke or spinal cord injury, often experience difficulties in movement. Those difficulties include but are not limited to freeze of gait, stiffness, slowness and tremors or shaking. This often impacts, sometimes severely, on the ability of such individuals to carry out normal daily activities. In some cases, this can lead to a loss of confidence, loss of independence and in some cases more serious conditions such as depression. Currently around 145,000 people in the UK have been diagnosed with Parkinson's disease, and it is estimated that there are approximately 10 million sufferers worldwide. Every hour, two people in the UK are diagnosed with Parkinson's disease. Around 2.3 million people worldwide suffer from MS. Approximately 15 million people suffer a stroke worldwide each year, and between 250,000 and 500,000 people suffer from a spinal cord injury worldwide each year. Additionally, approximately 68 million people worldwide have a stutter or stammer. Currently, approaches to dealing with the symptoms of such conditions typically revolve around medication or deep brain stimulation, both of which are complex and costly. Non-pharmacological and non-invasive approaches to addressing symptoms of such conditions would be preferred.

US 2012/259255 A1 represents the relevant background art.

The present invention has been devised with the foregoing in mind.

### SUMMARY

The present invention provides a wearable device according to claim 1. Further aspects and preferred embodiments are defined in the dependent claims. Aspects, embodiments, examples, and methods the present disclosure that do not fall within the scope of the appended claims do not form part of the invention and are merely provided for illustrative purposes.

According to a first aspect, there is provided a wearable device. The wearable device may be configured to alleviate or eliminate physical symptoms of neurological conditions such as Parkinson's disease. The wearable device may comprise at least one stimulating element configured to provide at least one mechanical stimulus to a user. The wearable device may also comprise a dissipating portion configured to increase an effective area of a mechanical stimulus provided by the at least one stimulating element.

According to a second aspect, there is provided a wearable device. The wearable device may be configured to alleviate or eliminate physical symptoms of neurological conditions such as Parkinson's disease. The wearable device may comprise at least one stimulating element configured to provide at least one mechanical stimulus to a user. The wearable device may be configured to provide a pulsed mechanical stimulus to a user. The wearable device may be configured to be attachable to, detachable from and/or reattachable to the skin of a user. The wearable device of the second aspect may comprise one or more of the features of the first aspect, and vice versa.

With reference to either of the above two aspects and/or to other aspects/embodiments below, sufferers of neurological conditions such as Parkinson's disease often experience freeze of gait, slowness, stiffness or tremors. Applying a mechanical stimulus (for example, a vibration, a pressure or change in pressure, a rolling motion, a tap or other impact etc.) can aid in alleviating or eliminating such symptoms. Increasing an effective area of the mechanical stimulus provided by the at least one stimulating element may enable the effect of the mechanical stimulus to be maximised. The application of the mechanical stimulus may therefore not be limited to specific local application reliant on direct contact with the at least one stimulating element. Rather, a surface area across which the mechanical stimulus may be applied may be increased significantly by providing a dissipating portion. This may reduce power requirements and/or reduce a number of stimulating elements required to provide a mechanical stimulus across a larger surface area of an individual. The wearable device of the present invention may be used by a person in addition to taking a prescribed medication, to help alleviate and manage any symptoms they are experiencing.

The dissipating portion may be or comprise a flexible, elastic or viscoelastic material. For example, a suitable material may be or comprise any one or more of silicone, rubber, flexible plastic, foam, metal, alloy of metal, etc.

The wearable device may comprise a surface configured to deliver or transmit a mechanical stimulus to the user. At least a part of the dissipating portion may be configured to form at least part of the surface configured to deliver or transmit a/the mechanical stimulus to the user. The at least one stimulating element may be configured to form at least part of the surface configured to deliver a/the mechanical stimulus to the user. This may enable different applications of the same mechanical stimulus provided by the at least one stimulating element. If only the dissipating portion is configured to form a part of the surface configured to deliver a mechanical stimulus to the user, the mechanical stimulus may be experienced by the user substantially consistently across an area corresponding to an area of the dissipating portion. If the at least one stimulating element also forms a part of the surface, the mechanical stimulus may be experienced in multiple ways by the user. In addition to the experience described above, the part of the surface formed by the stimulating element may be configured to deliver or transmit a/the mechanical stimulus to the user at a different intensity than the part of the surface formed by the dissipating portion. The stimulating element may be configured to deliver or transmit a/the mechanical stimulus to the user at a higher intensity than the dissipating portion. The user may experience a more intense local mechanical stimulus due to direct application of the mechanical stimulus from the at least one stimulating element. The combination of a local, intense mechanical stimulus and a less intense mechanical stimulus spread over a greater surface area may further improve the effect of the application of mechanical stimulus on freeze of gait, stiffness, slowness and tremors.

The at least one stimulating element may be or comprise a motor, for example a coin motor. The motor may be configured to provide a vibrational stimulus. Alternatively, the at least one stimulating element may comprise one or more rollers (for example, driven by a motor) or pressure elements, pistons etc. (for example, driven by actuators).

The dissipating portion may comprise a recess or aperture configured to receive the at least one stimulating element. The dissipating portion may at least partially surround or enclose the at least one stimulating element. The at least one stimulating element may be substantially flush with a mouth of the recess or aperture when the at least one stimulating element is received by the recess or aperture. In this way, the at least one stimulating element may form a part of the surface configured to deliver a mechanical stimulus to the user. The dissipating portion may be or comprise a substantially hollow shape (for example, a prismatic shape having a circular cross-section (a cylinder), a triangular cross-section, a square cross-section, a pentagonal cross-section or another polygonal cross-section). The dissipating portion may comprise a closed end and an open end to form a recess extending into the hollow shape from the open end. Alternatively, the dissipating portion may comprise two open ends to form an aperture extending between the ends of the dissipating portion.

A surface area of the dissipating portion (including the recess or aperture) may be significantly larger than a surface area of the at least one stimulating element, in order to increase an effective area of the mechanical stimulus provided by the at least one stimulating element. A more compact device may therefore be provided by locating the at least one stimulating element within a recess or aperture in the dissipating portion. The dissipating portion may also act to enclose and protect the at least one stimulating element if the at least one stimulating element is disposed within the dissipating portion.

The surface configured to deliver a mechanical stimulus to the user may comprise an adhesive configured to adhere the surface to skin of the user. Adhering the surface of the wearable device to skin of the user using an adhesive may provide numerous benefits. The adhesive may be provided on, or may be applicable to, the wearable device to make it ready for use. For example, using an adhesive may remove the need for any securing mechanism such as a clasp or buckle often utilised to affix a wearable device to the user. This may be particularly beneficial for users suffering from freeze of gait, stiffness, slowness or tremors. Additionally, the adhesive may improve a contact of the surface with skin of the user, improving an efficiency of delivery of the mechanical stimulus to the user. It also permits easy repositioning of the device in the event it is not correctly or optimally located initially. The wearable device may further comprise a removable and replaceable cover configured to cover the adhesive of the surface when the surface is not adhered to skin of the user. The cover may prevent an adhesive from drying out or becoming contaminated with foreign matter (for example, moisture, dust or other particles) when the wearable device is not adhered to skin of the user. The adhesive may be or comprise a medical grade adhesive. The adhesive may be configured to adhere the surface to skin of the user for an extended period of time, for example two or more days, such as three, four, five, six, seven, ten, most preferably 14 or more days. This may further improve ease of operation for the user, by reducing fixation and removal of the wearable device from skin of the user with the adhesive remaining on the skin. The same adhesive is used again for attaching the wearable device after being charged. Eventually when the adhesive has been used for the required number of days, for example 14 days or more, the adhesive can be replaced with a new layer of adhesive.

The wearable device may further comprise a charging portion (for example an electric charging portion). The charging portion may be configured to charge a rechargeable power source (such as a battery) of the wearable device. The charging portion may be configured to receive electric power wirelessly from an external electric power source (for example, using pin charging method, such as USB charging or any other appropriate method). The charging portion may be or comprise one or more coils or windings. The charging portion may be disposed in the recess of the dissipating portion. A charging portion configured to receive electric power wirelessly may further improve ease of operation for users suffering from freeze of gait, tremors, slowness or stiffness. Such a charging portion may remove the need for a wired charging connector to be physically connected to a charging port in the wearable unit in order to charge the wearable unit. Physically connecting charging components such as a connector to a port may be difficult for individuals suffering from freeze of gait, tremors, slowness or stiffness. A charging portion configured to receive electric power wirelessly may result in a simpler charging process, for example simply placing the wearable unit within a pre-determined distance of an external electric power source.

The wearable device may comprise a housing. The housing may be configured to at least partially surround the dissipating portion. The housing may be or comprise a plastic material (for example, polypropylene or polycarbonate) or a metal or alloy of metal (for example, aluminium). The housing may be configured to leave the surface configured to deliver a mechanical stimulus to the user exposed. The housing may be configured to surround an axially extending portion of the dissipating portion (for example, the housing may not be configured to cover end faces of the dissipating portion). The housing may protect the dissipating portion from impact, scratches or other degradation which could affect performance of the dissipating portion.

An output of the at least one stimulating element may be or comprise a pulsed output. The pulsed output may be or comprise a periodic output. A frequency of the periodic output may be between substantially 300 Hz and 0 Hz, and may be between substantially 200 Hz and 0 Hz, and may be between substantially 80 Hz and 0 Hz, and may be between substantially 40 Hz and 0 Hz, and may be between substantially 20 Hz and 0 Hz, and may be between substantially 20 Hz, and 0 Hz and may be between substantially 4 Hz and 0.25 Hz, and may be substantially 1 Hz. Applying a pulse-like mechanical stimulus may avoid the drawbacks of applying a constant or continuous mechanical stimulus that is too strong or too high in intensity (which may cause discomfort or irritation) or too weak or too low in intensity (which may have little effect on alleviation of physical symptoms such as freeze of gait, slowness, stiffness or tremors). A pulsed mechanical stimulus may also enable the frequency and intensity fluctuations of the output of the at least one stimulating element to provide cue signs to the user (for example, the pulsed mechanical stimulus may act as a metronome or timing indicator). A pulsed output comprising a regular, rhythmic or periodic fluctuation may modulate sensory dysfunction, and may enable users to focus on the cues provided by the pulsed output to coordinate timings of their own movement to the pulse-like mechanical stimulus. In doing so, freeze of gait, tremors, stiffness or slowness may be further reduced. Providing a tactile cue sign (using a mechanical stimulus) rather than, for example, visible light cue signs or auditory cue sounds may be beneficial. Visible light and auditory cue signs require a user to actively engage with the cue sign. Actively focusing on a cue sign may detract from concentration on a desired task, achieving the opposite to the intended effect. In contrast, the user may inherently be aware of the mechanical stimulus or tactile cue sign without requiring active focus, and so use of tactile cue signs may provide increased benefit to aid in improving movement and task completion.

Each period of the pulsed output comprises at least a first segment and a second segment. The first segment may comprise between substantially 75 % and 90 % of each period of the pulsed output. The first segment may comprise increasing an output intensity of the at least one stimulating element from a first intensity level to a second intensity level. The second segment may comprise decreasing an output intensity of the at least one stimulating element from the second intensity level to the first intensity level. The first intensity level may be substantially zero (no output from the at least one stimulating element). The increase in output intensity in the first segment may be substantially linear, or may be substantially non-linear. The decrease in output intensity in the second segment may be substantially linear, or may be substantially non-linear. A rate of increase in output intensity in the first segment may be different from a rate of decrease in output intensity in the second segment. The rate of increase in output intensity in the first segment may be lower than the rate of decrease in output intensity in the second segment. The rate of increase in output intensity in the first segment may be between substantially three and ten times lower than the rate of decrease in output intensity in the second segment. A duration of the first segment may be between three and ten times longer than the duration of the second segment. The difference in the relative durations of the first segment and the second segment of each period, and/or the difference in the rates of increase and decrease between the first segment and the second segment of each period, may contribute to and/or enhance the pulsed output experienced by the user to further reduce physical symptoms such as freeze of gait, tremors, stiffness or slowness.

The wearable device may further comprise a controller configured to control an output of the at least one stimulating element. The wearable device may also comprise a user input. The user input may be configured to receive at least one command from the user. The user input may be configured to relay the at least one command to the controller. In this regard, the wearable device may be configured with a to-use application. Such application may further be integrated with an external device, such as a smart phone, a tablet, a palmtop, etc. The user input may be disposed substantially opposite the surface configured to deliver a mechanical stimulus to the user. Disposing the user input substantially opposite the surface may enable easy, simple access to the user input when the wearable device is being worn (for example, when the surface configured to deliver a mechanical stimulus to the user is adhered to or otherwise in contact with skin of the user). This may be particularly beneficial for individuals suffering from freeze of gait, tremors, stiffness or slowness.

The user input may be configured to receive one or more commands to instruct the at least one stimulating element to provide different outputs. The user input may be configured to receive at least a first command to cause the controller to instruct the at least one stimulating to provide a first output. The user input may be configured to receive a second command, different from the first command, to cause the controller to instruct the at least one stimulating element to provide a second output. The first output may be or comprise at least one of a different length, frequency, intensity and change in intensity of a mechanical stimulus from the second output. For example, the first output may comprise a pulse-like mechanical stimulus provided for a pre-determined period of time (for example, between substantially one minute and five minutes, such as substantially two minutes), whilst the second output may comprise a substantially similar pulse-like mechanical stimulus provided continuously until the controller instructs the at least one stimulating element otherwise. The first output may be beneficial for aiding specific tasks which may be completed in a short period of time (for example, tying shoelaces, writing or typing short notes, brushing teeth, crossing the road). The user may not be required to proactively cease output of the at least one stimulating element in such cases for which the first output is suitable. The second output may be beneficial for aiding tasks which have a less definite length and are likely to last a longer period of time, for example, walking for a longer period of time (for example, five minutes or more, such as up to 15 minutes or up to 30 minutes), or writing or typing longer pieces of work.

According to a third aspect, there is provided a kit of parts. The kit of parts may comprise the wearable device of the first or second aspects. The kit of parts may comprise a docking station.

The docking station may comprise a recess configured to receive the wearable device (for example, when the wearable device is not being worn by a user). The docking station may support the wearable device when it is received within the recess. The docking station may be configured to transmit electric power wirelessly. The docking station may comprise a wireless charging transmitter. The docking station may comprise one or more coils or windings of electrically conductive material configured to wirelessly transmit electric power. The docking station may be configured to wirelessly transmit electric power to the wearable device. The docking station may be configured to wirelessly transmit electric power to the wearable device when the wearable device is received within the recess of the docking station. The docking station may be configured to connect to an external power source using a wired connection to provide electric power (for example, to the wireless charging transmitter) to be wirelessly transmitted to the wearable device.

The docking station may comprise at least a first portion and a second portion. The first portion may be or comprise a base. The second portion may be or comprise a lid or closure. The first portion and the second portion may be connected together form a housing or case for the wearable device. The housing or case may protect the wearable device during storage or transit. The first portion and the second portion may be releasably attachable, for example using corresponding male and female engagement features such as a press-fit or interference fit, elastic clips and flanges, or complementary screw threads. The first portion and the second unit may form an internal recess of the docking station to securely enclose the wearable device within the case when the first portion and the second portion are connected or attached together.

The first portion may comprise the recess configured to receive the wearable device. The first portion may comprise the wireless charging transmitter. The recess may be disposed in an outer surface of the first portion. The recess may be disposed in an upper surface of the first portion. A lower surface of the first portion may be substantially opposite the upper surface of the first portion. The first portion may rest on or stand on the lower surface in normal use. Moreover, the first portion may comprise a reservoir for containing additional adhesive for later use. The second portion may not be connected to the first portion in normal use (for example, when the wearable unit is received within the recess to receive electric power wirelessly from the docking station). The second portion may be connected to the first portion to store or transport the wearable device.

The docking station may comprise a reservoir of adhesive material. The adhesive material in the reservoir may be applied to the wearable device to attach the wearable device to skin of a user. The reservoir may be disposed in the first portion of the docking station. Alternatively, the reservoir may be disposed in the second portion of the docking station. An outer surface (for example, an end wall or a side wall) of the docking station may be replaceably removable from the docking station to provide access to the reservoir (for example, for the user). The reservoir may be refillable with adhesive material. A size of the outer surface replaceably removable from the docking station may be large enough to allow the wearable device (for example, a surface configured to deliver or transmit a mechanical stimulus to the user) to pass into the reservoir and contact the adhesive in the reservoir. This may enable easy application of additional adhesive to the wearable device as and when required.

According to a fourth, exemplary, aspect of the present disclosure, there is provided a method of applying a mechanical stimulus to the user. The method may comprise using the wearable device of the first or second aspect to apply a mechanical stimulus to the user. The method may be used to alleviate physical symptoms such as freeze of gait, stiffness, slowness or tremors (often exhibited by individuals suffering from neurological conditions), or to improve smoothness of speech in people suffering from speech impediments such as a stutter.

The method may comprise applying a mechanical stimulus to a sternum of the user. The method may comprise applying a mechanical stimulus behind an ear of the user. The method may comprise applying a pulsed mechanical stimulus to a user (for example, to a sternum of the user). The pulsed mechanical stimulus may be or comprise a periodic mechanical stimulus. A frequency of the periodic mechanical stimulus may be between substantially 4 Hz and 0.25 Hz, and may be substantially 1 Hz. Applying a pulse-like mechanical stimulus may avoid the drawbacks of applying a constant or continuous mechanical stimulus that is too strong or too high in intensity (which may cause discomfort or irritation) or too weak or too low in intensity (which may have little effect on alleviation of physical symptoms such as freeze of gait, slowness, stiffness or tremors). A pulsed mechanical stimulus may also enable the frequency and intensity fluctuations of the output of the at least one stimulating element to provide cue signs to the user (for example, the pulsed mechanical stimulus may act as a metronome or timing indicator). A pulsed output comprising a regular, rhythmic or periodic fluctuation may modulate sensory dysfunction, and may enable users to focus on the cues provided by the pulsed output to coordinate timings of their own movement to the pulse-like mechanical stimulus. In doing so, freeze of gait, tremors, stiffness or slowness may be further reduced. Providing a tactile cue sign (using a mechanical stimulus) rather than, for example, visible light cue signs or auditory cue sounds may be beneficial. Visible light and auditory cue signs require a user to actively engage with the cue sign. Actively focusing on a cue sign may detract from concentration on a desired task, achieving the opposite to the intended effect. In contrast, the user may inherently be aware of the mechanical stimulus or tactile cue sign without requiring active focus, and so use of tactile cue signs may provide increased benefit to aid in improving movement and task completion.

Optionally, there is provided a wearable device for modulating a sensory dysfunction or relieving a physical symptom associated with a neurological condition or disease. The wearable device comprises at least one stimulating element configured to provide at least one mechanical stimulus to a user, wherein the mechanical stimulus comprises a pulsed output; and a dissipating portion configured to increase an effective area of a mechanical stimulus provided by the at least one stimulating element, the dissipating portion coupled with the at least one stimulating element, wherein the wearable device, when in operation, is in physical contact with body of a user. The user may be suffering from a neurological condition or disease, for example Parkinson's disease, Alzheimer's disease, multiple sclerosis. In aforesaid diseases, the patient suffers from a neurological condition that affects their movement. In this regard, the wearable device is configured to enable the at least one stimulating element to generate pulsed output. Consequently, the pulsed output provides effective but gentle electrical (or vibrotactile) stimulations serving as cue signs for the user. The pulsed output comprises a regular, rhythmic or periodic fluctuation that may be generated as a continuous waveform or as binary mechanical stimulus. The binary mechanical stimulus provides a tactile cue sign, that enables modulation sensory dysfunction, and enables users to focus on the cues provided by the pulsed output to coordinate timings of their own movement to the pulsed output. In doing so, freeze of gait, tremors, stiffness or slowness, associated with the aforesaid medical conditions, may be further reduced. The tactile cue sign is beneficial to aid in improving movement and task completion as the user may inherently be aware of the mechanical stimulus or tactile cue sign without requiring active focus while being detracted from concentrating on a desired task.

The pulsed output is a balanced pulsed output. The balanced pulsed output results in symmetrical positive portion (cathodic pulse) characterized by high amplitude and negative portion (anodic pulse) characterized by very low amplitude, preferably zero, but with substantially equal pulse durations.

The pulsed output results in 6 to 50% improvement in the user, preferably 32% improvement. The improvement of up to 32% is observed for the activation of neurons as well as coordination of movement based on the pulsed stimulation. Moreover, the movement coordination is associated with user's focus on the cues provided by the pulsed output to coordinate timings of their own movement to the pulsed output. In doing so, freeze of gait, tremors, stiffness or slowness, associated with the aforesaid medical conditions, may be further reduced. The tactile cue sign is beneficial to aid in improving movement and task completion as the user may inherently be aware of the mechanical stimulus or tactile cue sign without requiring active focus while being detracted from concentrating on a desired task.

The wearable device makes the physical contact with body of the user by a mechanical engagement, and wherein the mechanical engagement is by any of: an adhesive, a strap, a locket, a bracelet, a band, a belt, a vacuum cup, a magnet. The physical contact with the body of the user, i.e. skin on the user, enables the transmission of the tactile stimulus from the wearable device to the user. The wearable device may be placed against any body part such as sternum, arm, shoulder, wrist, neck, ankle, leg and temple. The aforementioned body parts allow for easy access of the device by the user and by a carer or family member of an incapacitated user. The appealing design of the wearable device serves the device to be flaunted as an ornament. However, some users may be not be comfortable in showing off the wearable device and may wear the device at any other body part hidden under a piece of cloth for example. For example, the wearable device may be placed against thighs or on the back of the user.

The reservoir for adhesive is provided in the base of the docking station or the lid of the docking station, and wherein the reservoir rotatably removable from the base of the docking station or a lid of the docking station. The wearable device is attachable to the skin of the user, preferably sternum, by for example adhesive. The adhesive is provided as consumable pads of hook-and loop material comprising layers of adhesives on the back side thereof. A medical grade adhesive is provided at the back of the loop material that is placed facing towards the skin of the user, and a stronger adhesive is provided at the back of the hook material that is placed facing towards the wearable device, such as on the dissipating portion thereof. The hook and loop material attach together by way of hooks and loops and hold the wearable device in physical contact with body of the user. The consumable pads of adhesive are supplied and stored in layers in the reservoir for adhesive. The unique design of the reservoir enables accessing the stored consumable pads of adhesive when the consumable pads of adhesive presently in use with the wearable device is unable to show the desired effect. The rotating outwards of the reservoir enables keeping the consumable pads of adhesive in place and enhancing the longevity of the docking station by subjecting it to least amount of jerks and orientational changes.

The optional features from any aspect may be combined with the features of any other aspect, in any combination. For example, the device of the first or second aspect may comprise using a method that includes any of the features described with reference to the fourth aspect. Furthermore, the method of the fourth aspect may comprise any of the optional features described with reference to the device of the first or second aspect.

Features which are described in the context of separate aspects and embodiments of the invention may be used together and/or be interchangeable wherever possible. Similarly, where features are, for brevity, described in the context of a single embodiment, those features may also be provided separately or in any suitable sub-combination. Features described in connection with the method may have corresponding features definable with respect to the device and use of the device, and these embodiments are specifically envisaged.

### BRIEF DESCRIPTION OF THE DRAWINGS

The invention will now be described by way of example with reference to the accompanying drawings in which:
FIG. 1 shows a wearable device in accordance with an embodiment of the invention comprising a dissipating portion having a recess;
FIG. 2 shows a wearable device in accordance with an embodiment of the invention comprising a dissipating portion having an aperture;
FIG. 3 shows a wearable device in accordance with an embodiment of the invention comprising an adhesive;
FIGs. 4A and 4B show a wearable device in accordance with an embodiment of the invention comprising a charging portion;
FIGs. 4C and 4D show a unit or case for a wearable device in accordance with an embodiment of the invention;
FIGs. 4F, 4G and 4H show a unit or docking station in accordance with a preferred embodiment of the invention;
FIG. 4I shows a unit or docking station containing a wearable device in accordance with a preferred embodiment of the invention;
FIG. 4J shows a consumable adhesive pad in accordance with an embodiment of the invention;
FIG. 5 shows a wearable device in accordance with an embodiment of the invention comprising a controller to control an output of at least one stimulating element;
FIG. 6 shows an output of at least one stimulating element in accordance with an embodiment of the invention;
FIGs. 6A, 6B, 6C, 6D and 6E show graphs depicting duty cycle threshold determination for a wearable device in accordance with various embodiments of the invention
FIG. 7 shows another wearable device in accordance with an embodiment of the invention;
FIG. 8 shows a control scheme for operating a wearable device in accordance with an embodiment of the invention;
FIGs. 9A, 9B and 9C show another wearable device in accordance with an embodiment of the invention comprising a housing and an insert;
FIG. 10 shows a schematic illustration of a wearable device and docking station in accordance with an embodiment of the invention;
FIGs. 11A, 11B show schematic illustrations of a wearable device being configured for tracking by a mobile application software, when in operation in accordance with an embodiment of the invention; and
FIGs. 12A, 12B, 12C, 13A, 13B, 13C, 14A and 14B show schematic illustration of a user interface of a mobile application software in accordance with an embodiment of the invention.

Like reference numbers and designations in the various drawings indicate like elements.

### DETAILED DESCRIPTION

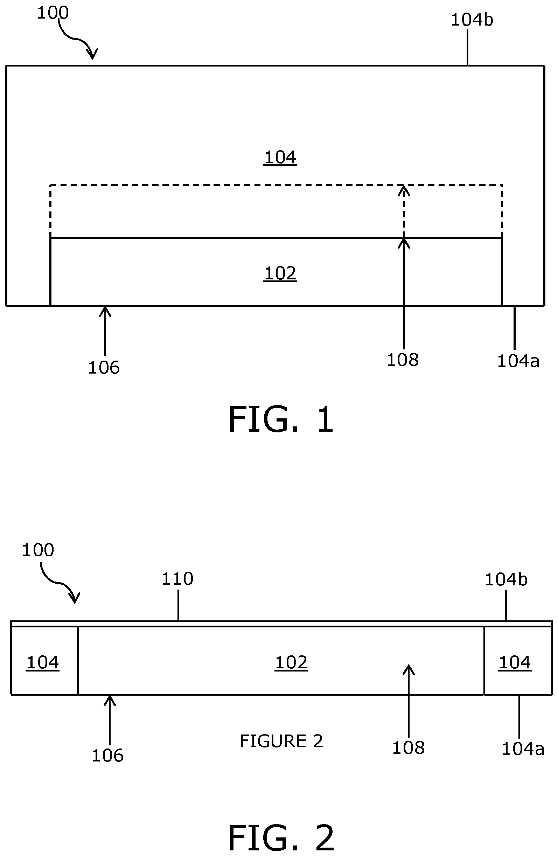

Figure 1 shows an embodiment of a wearable device 100 in accordance with the invention. The wearable device 100 comprises at least one stimulating element 102. The at least one stimulating element 102 is configured to provide at least one mechanical stimulus to a user of the wearable device 100. The wearable device 100 also comprises a dissipating portion 104. The dissipating portion 104 is configured to increase an effective area of a mechanical stimulus provided by the at least one stimulating element 102.

In the embodiment shown, the at least one stimulating element 102 is configured to provide a vibrational stimulus to a user of the wearable device 100, although it will be appreciated that mechanical stimuli provided by the at least one stimulating element may also or instead be or include a pressure or a change in pressure, a rolling motion, a tap or other impact etc. Notably, the mechanical stimuli or impacts are received as a force that affects muscles and neurons. Such impacts may be measured in Newton (N) units. In the embodiment shown, the at least one stimulating element 102 comprises a motor (such as a coin motor) configured to provide a vibrational stimulus to the user when the stimulating element is operational. In some embodiments, the motor is an electric motor for producing vibrotactile stimulation. In some embodiments, the output speed of the motor is between substantially 5000 RPM and 20000 RPM, for example substantially 12000 RPM. In some embodiments, a width or diameter of the motor is between substantially 5 mm and 20 mm, for example substantially 10 mm. In some embodiments, a thickness of the motor is between substantially 1 mm and 5 mm, for example substantially 2 mm. In some embodiments, the motor is arranged close to the skin of the user to provide a vibrational stimulus to the user when the stimulating element is operational. In some embodiments, the motor is surrounded with a layer of flexible or elastic material, such as silicone, and subsequently arranged to provide a vibrational stimulus to the user when the stimulating element is operational. Alternatively, the motor sits on a layer of flexible or elastic material, such as silicone, and subsequently arranged to contact the skin via the base layer of flexible or elastic material, to provide a vibrational stimulus to the user when the stimulating element is operational. Beneficially, the layer of flexible or elastic material, preferably silicone, reduces a noise (often disturbing) attributed to the motor and provides vibrational stimulus that is comfortable to the user.

The dissipating portion 104 is configured to increase an effective area of a mechanical stimulus provided by the at least one stimulating element 102 by transmitting the mechanical stimulus across a surface area larger than that of the at least one stimulating element 102. In some embodiments, the dissipating portion 104 comprises a flexible or elastic material. For example, a viscoelastic material, or a viscoelastic polymeric material such as a silicone, rubber, flexible plastic, foam, etc. may be used. The dissipating portion 104 forms at least a part of a (proximal) surface 106 of the wearable device 100 configured to contact (either directly or indirectly, for example via an adhesive, or a vacuum cup) skin of the user. The surface 106 is configured to deliver and/or transmit the mechanical stimulus from the stimulating element 102 to the user. In the embodiment shown, the at least one stimulating element 102 also forms at least a part of the surface 106. In alternative embodiments, the at least one stimulating element 102 may not form (for example, may be set back from) a part of the surface 106. The stimulating element 102 may be provided or housed in the dissipating portion 104. In the embodiment shown, the dissipating portion 104 is in direct contact with the at least one stimulating element 102. In alternative embodiments, the dissipating portion 104 is in indirect contact with the at least one stimulating element, for example via one or more other components of the wearable device 100. In some embodiments, a diameter or width of the dissipating portion 104 is between substantially 10 mm and 40 mm. In some embodiments, the dissipating portion 104 has or comprises a thickness of at least 3 mm. In some embodiments, the dissipating portion 104 has or comprises a thickness of at least 3 mm in all dimensions of the dissipating portion 104 not comprising a recess or aperture 108. For example, in all directions surrounding the at least one stimulating element 102, the dissipating portion 104 has a thickness of between 3 mm and 10 mm.

In the embodiment shown, the dissipating portion 104 is or comprises a substantially cylindrical shape having a first (or proximal) end 104a and a second (or distal) end 104b. "Proximal" refers to the end that is placeable on the user's skin and "distal" refers to the opposite or outmost end. It will be appreciated that the dissipating portion 104 may have or comprise another shape (for example, a triangular, square, pentagonal or other polygonal prismatic shape). The first end 104a forms at least a part of the surface 106 configured to contact skin of the user. In the embodiment shown, the first end 104a of the dissipating portion 104 comprises a recess 108. The at least one stimulating element 102 is configured to be received within the recess 108. In the embodiment shown, the at least one stimulating element 102 substantially fills the recess 108 in all directions (as indicated by the solid lines indicating the recess 108, such that the at least one stimulating element 102 is substantially the same size as the recess 108). In this way, the dissipating portion 104 is configured to surround the at least one stimulating element 102 except for at the first end 104a, where the at least one stimulating element 102 is left exposed. In alternative embodiments, the at least one stimulating element 102 is smaller than the recess 108 (as indicated by the dashed lines indicating the recess 108, such that the recess 108 comprises additional depth not filled by the at least one stimulating element 102, although it will be appreciated that the recess 108 may comprise one or both of additional depth and additional width relative to the at least one stimulating element 102). In such embodiments, the recess 108 is configured to be substantially filled by a combination of the at least one stimulating element 102 and one or more other components of the wearable device 100 (for example, a power source such as a battery configured to power the at least one stimulating element 102). In the embodiment shown, the recess 108 has or comprises a substantially similar shape to the shape of the dissipating portion 104, although it will be appreciated that the recess 108 may have or comprise another shape (for example, a shape substantially similar to a shape of the at least one stimulating element 102 and/or a shape of one or more other components of the wearable device 100). In an embodiment, the stimulating element 102 may have a tight interference fit within the recess 108, at least in the lateral direction (i.e. substantially perpendicular to the proximal-distal direction). Additionally or alternatively, there may not be a tight interference fit in any/all directions and other means may be provided to make or form a connection between the stimulating element 102 and the dissipating portion 104.

In the embodiment shown, the at least one stimulating element 102 is substantially flush with a mouth of the recess 108 when the at least one stimulating element 102 is received within the recess 108, in order to form (at least a part of) the surface 106. In alternative embodiments, the at least one stimulating element 102 is set back from the mouth of the recess 108 and does not form a part of the surface 106 (although the dissipating portion 104 ensures that the mechanical stimulus is still spread across an effective area larger than the at least one stimulating element 102). In the embodiment shown, the second end 104b comprises a closed end of the substantially cylindrical shape of the dissipating portion 104. The second end 104b is configured to form an outer or distal surface of the wearable device 100.

In alternative embodiments, such as shown in Figure 2, the second end 104b is open, and the recess 108 extends through the dissipating portion 104 to the open second end 104b to form an aperture (e.g., a recess open at both ends) through the dissipating portion 104. In some such embodiments, an additional capping layer 110 is provided to ensure the at least one stimulating element 102 and any other components of the wearable device 100 remain secured or located within the recess or aperture 108. In alternative embodiments, no capping layer is provided. The at least one stimulating element 102 and one or more other components of the wearable device 100 may be secured within the aperture 108 using, for example, a friction fit (such that the components of the wearable device 100 contact each other sufficiently closely, and with enough force, within the recess or aperture 108 to prevent inadvertent escape of one or more components of the wearable device 100 from the aperture or recess 108), or an adhesive. The capping layer 110 is configured to form an outer surface of the wearable device 100. In some embodiments, the capping layer 110 is or comprises the same material as the dissipating portion 104.

In some embodiments, the proximal surface 106 formed at least in part by the dissipating portion 104 (and optionally the at least one stimulating element 102) is configured to directly contact skin of the user. For example, in some embodiments the wearable device 100 is incorporated into one of a bracelet, a pendant, an anklet, an arm band, a wrist band or other item configured to be worn by a user such that the surface 106 is arranged to contact skin of the user when the item is worn by the user. The item worn by the user is configured to retain the wearable device 100 on skin of the user and maintain a contact pressure between the surface 106 of the wearable device 100 and skin of the user.

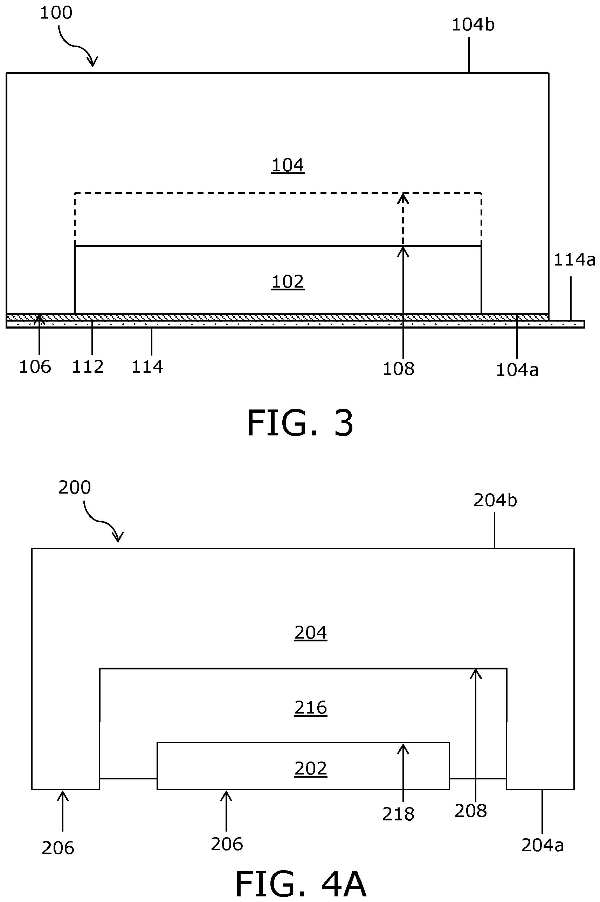

In alternative embodiments, such as shown in Figure 3, the proximal surface 106 comprises or is provided with an adhesive 112 (for example, a layer of adhesive) configured to adhere the surface 106 to skin of the user. In some such embodiments, the adhesive 112 may be or comprise a medical grade adhesive. A suitable adhesive, for example, is DuploMED ELE77301. In some embodiments, the adhesive 112 is configured to bond the surface 106 to skin of the user for an extended period of time, for example up to 7 days. In some embodiments, the adhesive 112 is at least one of waterproof, sweatproof and breathable in order to maintain comfort and cleanliness for the user. In some embodiments, the adhesive 112 is at least one of water resistant, sweat resistant in order to maintain comfort and cleanliness for the user. Beneficially, the waterproof and sweatproof adhesive enables use of the wearable devices in high moisture conditions such as, for example, during bathing, water activities and so forth. Additionally, the adhesive 112 is suitable for use by elderly patients whose skin is more fragile and sensitive with age. In some embodiments, the waterproof and sweatproof adhesive enables patient care, such as wet wipe off, for patients who are incapacitated. The adhesive 112 is durable and reduces the need to change the adhesive 112 frequently. Moreover, the water resistant and sweat resistant property of the adhesive 112 enables use of the wearable devices continuously for 14 days or more. The breathable adhesive is a medical grade adhesive that is skin-friendly and prevents potential irritation associated with an extended exposure of the user's skin to the adhesive.

In some embodiments, the wearable device comprises a cover 114 configured to cover and protect the adhesive 112 when the wearable device 100 is not adhered to the user. The cover 114 may be or comprise an acrylic film or layer. The cover 114 is configured to be removable from and replaceable on the adhesive 112 (for example, by peeling the cover 114 away from the adhesive 112). In some embodiments, the cover 114 comprises a tab 114a enabling the user to easily take hold of the cover 114 to remove the cover 114 from the adhesive 112 and/or replace the cover 114 on the adhesive 112. In some embodiments, the adhesive 112 is configured to seal the wearable device 100 and aid in retaining components of the wearable device 100 within the dissipating portion 104. In some embodiments, the adhesive 112 is disposed (at least in part) on a soft (for example, easily compressible) layer of material configured to be received within the recess 108. In some embodiments, the soft layer of material is configured to form at least a part of the surface 106. In some embodiments, the soft layer of material is used to ensure the surface 106 is a flat surface for the adhesive 112 to be applied to or disposed on. The soft layer of material fills any gaps or breaks in the surface between, for example, the dissipating portion 104 and the at least one stimulating element 102. For example, if the at least one stimulating element 102 is set back from the first end 104a or the recess 108 of the dissipating portion 104 (such that the at least one stimulating element 102 does not form a part of the surface 106 configured to contact skin of the user and deliver a mechanical stimulus to the user), the soft layer of material covers or surrounds the at least one stimulating element 102 to provide a substantially constant, level surface (for example, level with a mouth of the recess 108 of the dissipating portion 104) for the adhesive 112 to be disposed on or applied to. Alternatively, if the at least one stimulating element 102 is flush with a mouth of the recess but does not extend across a diameter of the recess 108, the soft layer of material may surround the at least one stimulating element 102 (for example, substantially filling the space around the at least one stimulating element 102 in the recess 108) to provide a substantially constant, level surface for the adhesive 112 to be disposed on or applied to. The soft layer of material may be or comprise urethane (for example, medical urethane), rubber, silicone or another flexible material. The soft layer of material may improve comfort of the wearable device 100 for the user when the surface 106 is in contact with skin of the user. The soft layer of material may not be permanently disposed in the recess 108, and may be removable or replaceable (for example, in between sequential wearings of the wearable device 100 by the user). The soft layer of material may be coated in adhesive 112 prior to being inserted into the recess 108. The soft layer of material may be provided as part of an insert comprising the soft layer of material, the adhesive 112 and the cover 114. The adhesive 112 may be sandwiched between the cover 114 and the soft layer of material. The soft layer of material may be inserted into the recess 108 to surround or cover the at least one stimulating element 102, forming a substantially level surface 106. The cover 114 may then be removed to expose the adhesive 112 in order to adhere the wearable device 100 to skin of the user.

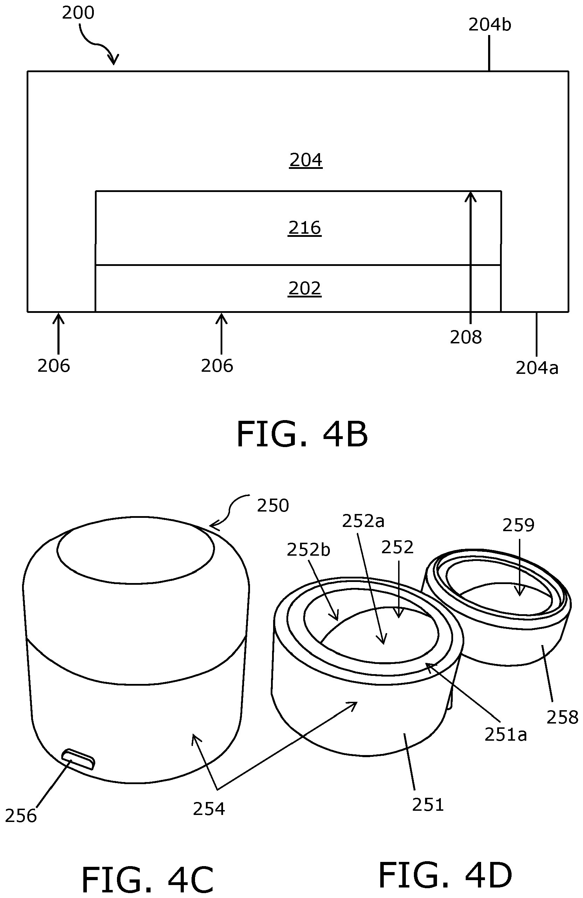

Figures 4A and 4B show embodiments of a wearable device 200 in accordance with the invention. The wearable device 200 comprises substantially similar features to those described with respect to the wearable device 100 above (like or corresponding reference numerals are used where relevant). The wearable device 200 comprises an electric charging portion 216. The charging portion 216 is configured to receive electric power in order to provide electrical power to power the at least one stimulating element 202 (either directly, or indirectly via a power source of the wearable device 200 such as a battery which the charging portion 216 is configured to charge). In the embodiment shown, the charging portion 216 is configured to receive electric power wirelessly from an external electric power source. In some embodiments, the charging portion 216 is or comprises one or more coils or windings configured to receive electric power wirelessly from an external power source (such as one or more powered coils or windings). In some embodiments, the one or more coils or windings may be or comprise Litz wire or copper wire. In some embodiments, the charging portion 216 is configured to receive electric power from an external electric power source via a wired connection (such as pin charging or any other appropriate method).

In the embodiments shown, the charging portion 216 is configured to be received or located within the recess 208. In the embodiment shown in Figure 4A, the charging portion 216 at least partially surrounds the at least one stimulating element 202 within the recess 208. The charging portion 216 comprises a recess 218 configured to at least partially receive the at least one stimulating element 202. In alternative embodiments, the recess 218 extends through a thickness of the charging portion 216 to form an aperture extending through the charging portion 216. In such embodiments, the at least one stimulating element 202 is at least partially received within the recess or aperture 218. In some embodiments, the charging portion 216 is contained within the recess 208 of the dissipating portion 204 such that the charging portion 216 does not form a part of the surface 206. That is, the charging portion 216 is set back from the first end 204a of the dissipating portion 204 when received within the recess 208. As such, in some embodiments such as shown in Figure 4A, the at least one stimulating element 202 sits proud of (e.g., not flush with) the charging portion 216. In alternative embodiments, such as shown in Figure 4B, the charging portion 216 does not comprise a recess configured to at least partially receive the at least one stimulating element 202. Rather, both the charging portion 216 and the at least one stimulating element 202 are received within the recess 208 without overlapping with one another. In some embodiments, the charging portion 216 and the at least one stimulating element 202 are arranged within the recess 208 to be layered on top of one another. The at least one stimulating element 102 is disposed nearer to the mouth of the recess 208 than the charging portion 216 is. In the embodiment shown, the at least one stimulating element 202 forms a part of the surface 206, but in alternative embodiments, the at least one stimulating element 202 is set back from the first end 204a of the dissipating portion 204. In the embodiment of Figure 4A or 4B, the stimulating element 202 is either not in direct contact with, or is only partially in direct contact with, the dissipating portion 204. However, stimulation can still be dissipated and transferred through the charging portion 216. It will be appreciated that the spatial arrangement of components of the wearable device 200 within the recess 208 may be arranged differently to the arrangement described above. In some embodiments, the charging portion 216 comprises a shape and/or is arranged within the recess such that it occupies a maximal space within the recess 208, whilst still enabling other components of the wearable device 200 to be disposed within the recess 208. A maximal volume of the charging portion 216 may maximise or increase battery life of the wearable device 200.

In a preferred embodiment, the charging portion 216 is configured to receive electric power from an external electric power source via a wired connection. In this regard, the wearable device 200 comprises a charging slot (not shown) having a receiving groove for receiving a charging head of a wired connector connected on the other end to the external electric power source. The receiving groove of the charging slot is suitably sized and shaped to accommodate a charging head of a wired connector having a size and a shape complimentary to the receiving groove. In this regard, the receiving groove of the charging slot and the charging head of the wired connector serve as lock and key. The charging head may have various shapes and sizes including, but not limited to, a cylindrical pin of various sizes, a USB, or a universal AC-DC connector, In one embodiment, the charging slot is shaped to receive a wired connector of a USB form factor, for example, a USB-A connector, a USB-B connector or a USB-C connector. In another embodiment, the charging slot is shaped to receive a wired connector of a pin form, for example a POGO PIN connector. In yet another embodiment, the charging slot is shaped to receive a wired connector of a 2-pin charging type.

In some embodiments, the wearable device 200 is charged at a charge rate in a range of 53 milliampere (mA) to 110 milliampere (mA) when a voltage of 5V is applied thereto. The 5V is a direct current voltage supplied by a battery or electric power source in the electronic circuit of the wearable device 200. In some embodiment, the voltage is in a range of 2.5 to 5V, optionally, 2.9 to 3V. In some embodiment, the voltage is 3V. In some embodiment, the voltage is in a range of 2.5 to 5V. Moreover, at such voltage conditions, the LEDs are driven to a maximum current of 5 mA. It will be appreciated that this brightness may not be visible enough through the light guide. A power regulation functioning of the wearable device 200 provides a measure of 2.999V on an output of a 3V3 regulator. In some embodiments, the wearable device 200 is designed for a dual polarity charging. In some embodiments, in addition to all the different types of charging slots (or ports), the wearable device 200 comprises a battery port and a programming port.

In some embodiments, the components of the wearable device 200 are suitable for a desired temperature and humidity. In some embodiments, the wearable device 200 has moisture sensitivity level (MSL) of 1 and the operating temperature is in a range between 0 °C and 125 °C. In some embodiments, the wearable device 200 has moisture sensitivity level (MSL) of 1 and the operating temperature is in a range between -40 °C and 85 °C. In some embodiments, the wearable device 200 has moisture sensitivity level (MSL) of 1 and the operating temperature is in a range between -40 °C and 125 °C.

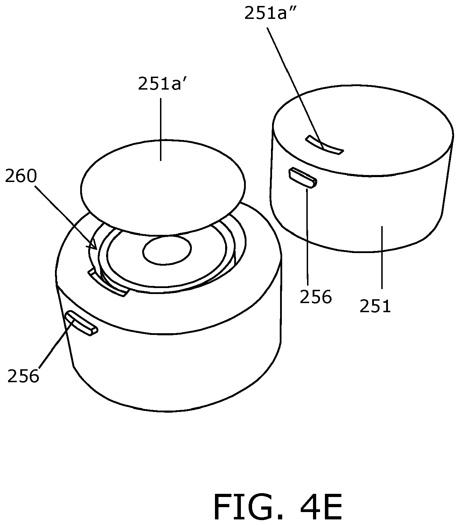

Figures 4C, 4D and 4E show a unit or docking station 250. The unit 250 is configured to hold and retain the wearable device 200 when the wearable device 200 is not being worn by the user. In the embodiment shown, the unit 250 comprises a recess 252 configured to receive the wearable device 200. The recess 252 comprises a receiving surface 252a on which the wearable device 200 is configured to be placed. The recess 252 also comprises a surrounding wall 252b configured to prevent movement of the wearable device 200 on the receiving surface 252a. In the embodiment shown, the surrounding wall 252b differs in height relative to the receiving surface 252a around a perimeter of the receiving surface 252a. The surrounding wall 252b is primarily disposed continuously on one half of the perimeter of the receiving surface 252a. In the embodiment shown, the receiving surface 252a is inclined towards the surrounding wall 252b at an acute angle (less than 90°, for example between substantially 15° and 25°). The angle between the receiving surface 252a and the surrounding wall 252b helps to retain the wearable device 200 on the receiving surface 252a, and prevents the wearable device 200 from inadvertently falling off the receiving surface 252a. In the embodiment shown, a wireless charging transmitter 254 (not shown, but location illustrated) of the unit 250 is disposed beneath the recess 252 within the unit 250. The wireless charging transmitter 254 is configured to wirelessly transmit electrical power to the charging portion 216 of the wearable device 200. In some embodiments, the wireless charging transmitter 254 is or comprises one or more coils or windings of electrically conductive material. The unit 250 further comprises a power connection 256 configured to connect the unit 250 to an external power source (for example, mains electricity) to provide power to the wireless charging transmitter 254. The power connection 256 is configured to receive a wired connection to the external power source. As such, connecting the unit 250 to the external power source, such as mains electricity, provides power to the wireless charging transmitter 254 which can wirelessly charge the charging portion 216.

In the embodiment shown, the recess 252 is disposed in an outer surface 251a of a first portion or base 251 of the unit 250. In normal use, the outer surface 251a may be an upper surface of the first portion 251, such that the first portion or base 251 supports the wearable device 200 when it is received in the recess 252. The power connection 256 is disposed on the first portion 251. In some embodiments, the unit 250 further comprises a second portion 258. The second portion 258 effectively is or comprises a lid or cover configured to close the recess 252 of the first portion 251. The second portion 258 and the first portion 251 are releasably attachable to one another, for example using corresponding male and female engagement features, such as a press-fit or friction fit, elastic clips and flanges or complementary screw threads. The second portion 258 comprises a recess 259 which is complementary to the recess 252 of the first portion 251. Together, when the second portion 258 is attached to the first portion 251, the recess 259 and the recess 252 form an internal recess of the unit 250 which is configured to securely enclose the wearable device 200 within the internal recess (for example, to prevent movement of the wearable device 200). As such, the unit 250 is configured to act as a case for the wearable unit 200, for example during storage or transit.

In some embodiments, the first portion 251 of the unit 250 comprises a reservoir 260 (shown in Figure 4E), within the first portion 251, in which additional adhesive for adhering the wearable device 200 to skin of the user may be contained or stored. In some embodiments, a part of the outer surface 251a' (for example, an end wall or a side wall) of the first portion 251 is removable (replaceably) from the first portion 251 to provide access to the reservoir 260. In some embodiments, the outer surface 251a comprises a notch 251a" enabling force to be applied to the part of the outer surface 251a' in order to remove it from the first portion 251. In some embodiments, the reservoir 260 is refillable. The additional adhesive may be provided to the reservoir 260 in distinct, separate layers of adhesive (for example, a stack of adhesive layers) which are individually retrievable from the reservoir 260 and which may be applied separately to the wearable device 200.

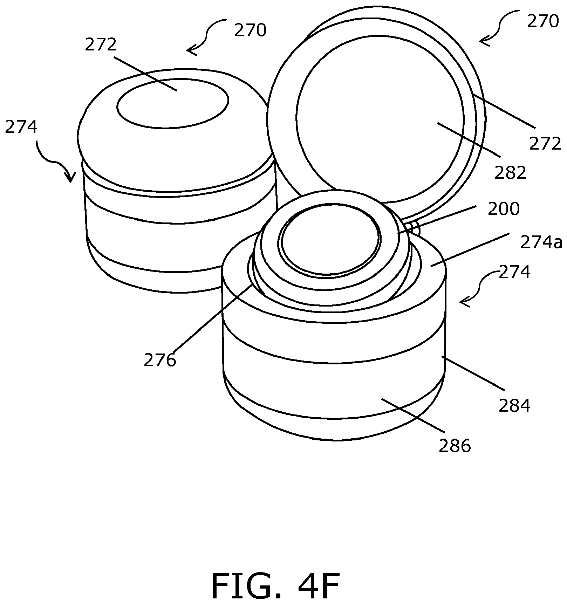

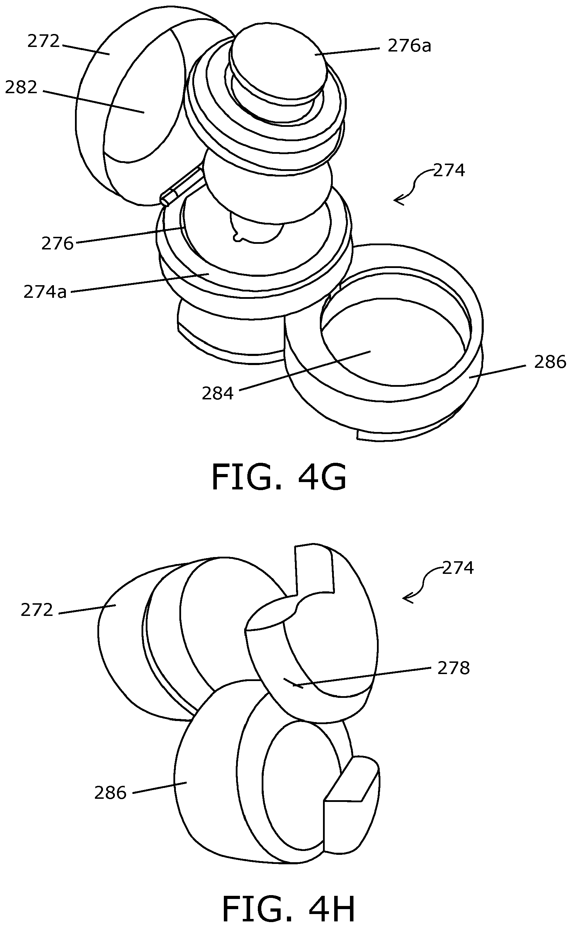

Figures 4F, 4G and 4H show a unit or docking station 270 in accordance with a preferred embodiment of the invention. The unit 270 is configured to hold and retain the wearable device 200 when the wearable device 200 is not being worn by the user. In the embodiment shown, the unit 270 comprises a single body having a lid 272 (referred to as a second portion hereafter) attached at one end to a base 274 (referred to as a first portion hereafter). As shown, the unit 270 comprises a recess 276 (shown in Figure 4G) configured to receive the wearable device 200. The recess 276 comprises a receiving surface 276a (shown in Figure 4G) on which the wearable device 200 is configured to be placed. The recess 276 also comprises a surrounding wall (not shown) configured to prevent movement of the wearable device 200 on the receiving surface 276a. In the embodiment shown, the surrounding wall differs in height relative to the receiving surface 276a around a perimeter of the receiving surface 276a. The surrounding wall is primarily disposed continuously on one half of the perimeter of the receiving surface 276a. In the embodiment shown, the receiving surface 276a is inclined towards the surrounding wall at an acute angle (less than 90°, for example between substantially 15° and 25°). The angle between the receiving surface 276a and the surrounding wall helps to retain the wearable device 200 on the receiving surface 276a, and prevents the wearable device 200 from inadvertently falling off the receiving surface 276a. In some embodiments, the receiving surface 276a comprises a small magnet (not shown). The magnet in the receiving surface 276a helps to retain the wearable device 200 on the receiving surface 276a. It will be appreciated that the wearable device 200 comprises various components that are potentially magnetic and are attracted to an opposite pole of another magnet, such as the magnet in the receiving surface 276a. The unit 270 further comprises a power connection 278 (or charging slot) configured to connect the unit 270 to an external power source (for example, mains electricity) to provide power to the wearable device 200. In some embodiments, the unit 270 is further configured to charge the wearable device wirelessly. In this regard, the unit 270 may be configured to wirelessly transmit electric power to the wearable device 200. The unit 270 may be configured to wirelessly transmit electric power when the wearable device 200 is received within the recess 276 of the unit 270. The unit 270 may comprise a wireless charging transmitter, such as one or more coils or windings of electrically conductive material, configured to wirelessly transmit electric power.

In the embodiment shown, the recess 276 is disposed in an outer surface 274a of a first portion (namely, base) 274 of the unit 270. In normal use, the outer surface 274a may be an upper surface of the first portion 274, such that the first portion 274 supports the wearable device 200 when it is received in the recess 276. The power connection 278 is disposed on the first portion 274. The second portion (namely lid) 272 of the unit 270 is configured to close the recess 276 of the first portion 274. The second portion 272 and the first portion 274 are connected to each other at an end 280, such that the second portion 272 and the first portion 274 are releasably attachable to one another, for example using corresponding male and female engagement features, for example fasteners, such as a clamp fastener, to enable access to the recess 276. The second portion 272 comprises a recess 282 which is complementary to the recess 276 of the first portion 274. Together, when the second portion 272 is attached to the first portion 274, the recess 282 and the recess 276 form an internal recess of the unit 270 which is configured to securely enclose the wearable device 200 within the internal recess (for example, to prevent movement of the wearable device 200). The second portion 272 and the first portion 274 securely enclose the wearable device 200 by attaching with each other by pieces of magnet at the closing ends thereof. As such, the unit 270 is configured to act as a case for the wearable unit 200, for example during storage or transit.

In the embodiment shown, a reservoir 284 for containing or storing additional adhesive is disposed in the unit 270. The additional adhesive in the reservoir 284 is available for application on to the wearable device 200 and on the user's skin for attaching the wearable device 200 to the skin of a user when in use. As shown in the illustration, the reservoir 284 is disposed in the first portion 274 of the unit 270. In such embodiment, a part of an outer surface 286 (for example, an end wall or a side wall) of the unit 270, containing the reservoir 284, is replaceably removable from the unit 270 to provide access to the reservoir 284. The reservoir 284 is replaceably removable by rotating outwards the part of the outer surface 286 containing the reservoir 284. The size of the reservoir 284 is large enough to allow the wearable device 200, for example, a surface (not shown) thereof configured to deliver or transmit a mechanical stimulus to the user, to pass into the reservoir 284 and contact the additional adhesive in the reservoir 284. Thereby enabling easy application of additional adhesive to the wearable device 200 as and when required. In some embodiments, the reservoir 284 is refillable with additional adhesive. In the embodiment shown, the reservoir 284 contains or stores additional adhesive in the form of one or more consumable adhesive pads 288 (shown in Figure 4G). The one or more consumable adhesive pads 288 are provided to the reservoir 284 in distinct, separate layers (for example, a stack of adhesive pads 288) which are individually retrievable from the reservoir 284 and which may be applied individually (or separately) to the wearable device 200. The one or more consumable adhesive pads 288 are arranged in the reservoir 284 such that the side corresponding (or attachable) to the surface of the wearable device 200 is exposed from the reservoir 284. When the wearable device 200 is pressed in the reservoir 284, a consumable adhesive pad 288 (a top layer) is transferred on to the surface of the wearable device 200. The other (or bottom layer) consumable adhesive pads 288 are retained within the reservoir 284 during this process. The consumable adhesive pad 288 comprises a peelable cover with a tab to enable easy removal of the cover.

In some embodiments, the reservoir 284 is disposed in the second portion 272 of the unit 270. Similarly, for the placement of the one or more consumable adhesive pads 288 in the reservoir of the first portion 274, the one or more consumable adhesive pads 288 are arranged in the second portion 272.

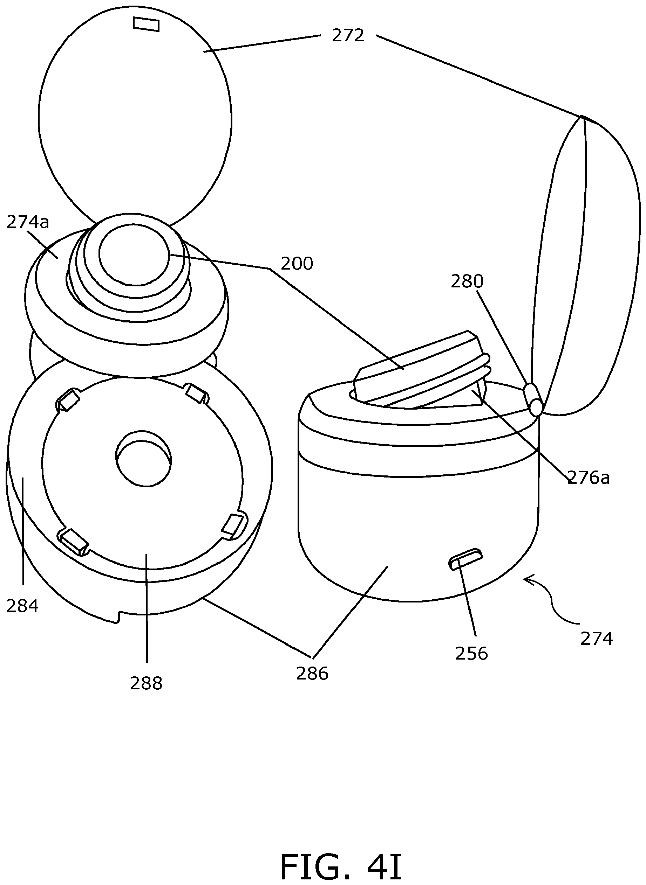

Figures 4I shows a unit or docking station 270 containing the wearable device 200, in accordance with a preferred embodiment of the invention. The unit 270 is configured to hold and retain the wearable device 200 when the wearable device 200 is not being worn by the user.

In the embodiment shown, the unit 270 comprises a single body having a lid 272 (referred to as a second portion hereafter) attached at one end to a base 274 (referred to as a first portion hereafter), as described above. As shown, wearable device 200 is received in a recess 276 of the unit 270. Specifically, the wearable device 200 is placed on a receiving surface 276a in the recess 276. More specifically, the receiving surface 276a is inclined and surrounded by a surrounding wall (not shown) configured to prevent movement of the wearable device 200 on the receiving surface 276a. The second portion (namely lid) 272 of the unit 270 is configured to close the recess 276 of the first portion 274. The second portion 272 and the first portion 274 are connected to each other at an end 280, such that that second portion 272 and the first portion 274 are releasably attachable to one another, for example using corresponding male and female engagement features, for example fasteners, such as clamp fastener, to enable access to the recess 276. The second portion 272 comprises a recess 282 which is complementary to the recess 276 of the first portion 274. Together, when the second portion 272 is attached to the first portion 274, the recess 282 and the recess 276 form an internal recess of the unit 270 which is configured to securely enclose the wearable device 200 within the internal recess (for example, to prevent movement of the wearable device 200). The second portion 272 and the first portion 274 securely enclose the wearable device 200 by attaching with each other by pieces of magnet at the closing ends thereof. As such, the unit 270 is configured to act as a case for the wearable unit 200, for example during storage or transit. The wearable device 200 receives a layer of adhesive from a reservoir 284 for containing or storing additional adhesive disposed in the unit 270. Moreover, a part of an outer surface 286 of the unit 270, containing the reservoir 284, is replaceably removable from the unit 270, by rotating outwards, to provide access to the reservoir 284. The size of the reservoir 284 is large enough to allow the wearable device 200, for example, a surface (not shown) thereof configured to deliver or transmit a mechanical stimulus to the user, to pass into the reservoir 284 and contact the additional adhesive in the reservoir 284. Thereby enabling easy application of additional adhesive, in the form of one or more consumable adhesive pads 288, to the wearable device 200 as and when required.

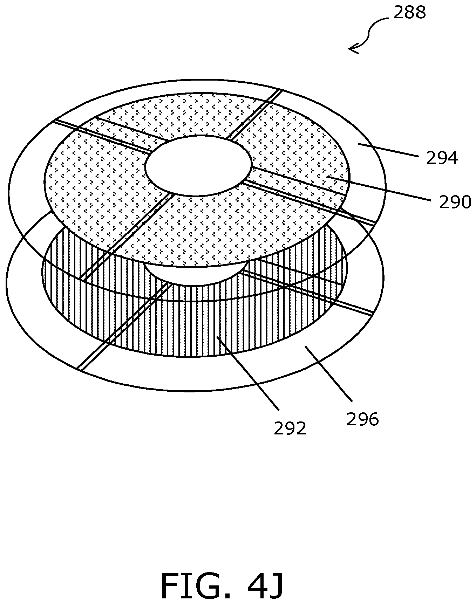

Figure 4J shows a consumable adhesive pad 288. The consumable adhesive pads 288 comprises a hook-and-loop fastener, such as a Velcro^{™}, possessing adhesive. The hook-and-loop fasteners comprise a hook layer 290 and a loop layer 292. The hook layer 290 and the loop layer 292 are configured to couple to each other by means of tiny hooks in the hook layer 290 and smaller loops in the loop layer 292. When the hook layer 290 and the loop layer 292 are pressed together the hooks catch in the loops and the two layers fasten or bind temporarily. The two layers are separated by pulling or peeling apart the loop layer 292 from the hook layer 290. The respective backs of the hook layer 290 and the loop layer 292 comprise adhesives. Adhesives on the hook layer 290 and the loop layer 292 are of different grades and strength. As shown, the consumable pad 288 comprises a first layer of adhesive 294 coupled to the hook layer 290 and a second layer of adhesive 296 coupled to the loop layer 292. The consumable adhesive pad 288 is arranged to attach to the skin of the user by the second layer of adhesive 296 coupled to the loop layer 292 and to the surface of the wearable device 200 the first layer of adhesive 294 coupled to the hook layer 290. As such the second layer of adhesive 296 is a medical grade adhesive for binding with the skin of the user, and the first layer of adhesive 294 is a stronger adhesive for binding with the wearable device 200.

The wearable device 200 can be placed onto the skin of the user using the consumable adhesive pad 288. The wearable device 200 can be removed for charging or when not required for use, with the loop layer 292 of the consumable adhesive pad 288 remaining attached on the skin via the second layer of adhesive 292, while hook layer 290 of the consumable adhesive pad 288 remains attached on the wearable device 200 via the first layer of adhesive 294. This allows using the same consumable adhesive pad 288, for a predefined time, while the wearable device 200 is being charged. Eventually when the consumable adhesive pad 288 has been used for the required number of days, for example 14 days or more, such as 20 days, the consumable adhesive pad 288 can be removed and a new consumable adhesive pad 288 can be applied using the process described above. Alternatively, the consumable adhesive pad 288 may be replaced if the consumable adhesive pad 288 fails to provide desired adhesion on the skin of the user or the wearable device 200. Moreover, the hook layer 290 is strong enough to last several months if not years. However, the hook layer 290 can also be detached from the wearable device 200 in case the hook layer 290 wears off.

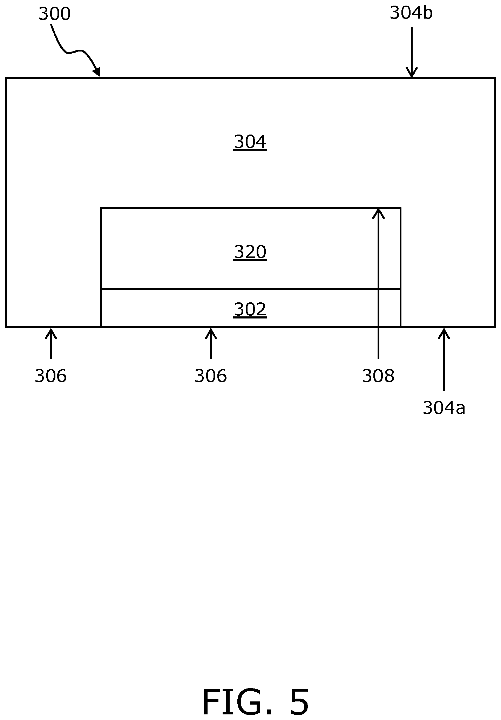

Figure 5 shows an embodiment of a wearable device 300 in accordance with the invention. The wearable device 300 comprises substantially similar features to those described with respect to the wearable device 100 and the wearable device 200 above (like or corresponding reference numerals are used where relevant). The wearable device 300 comprises a controller 320.

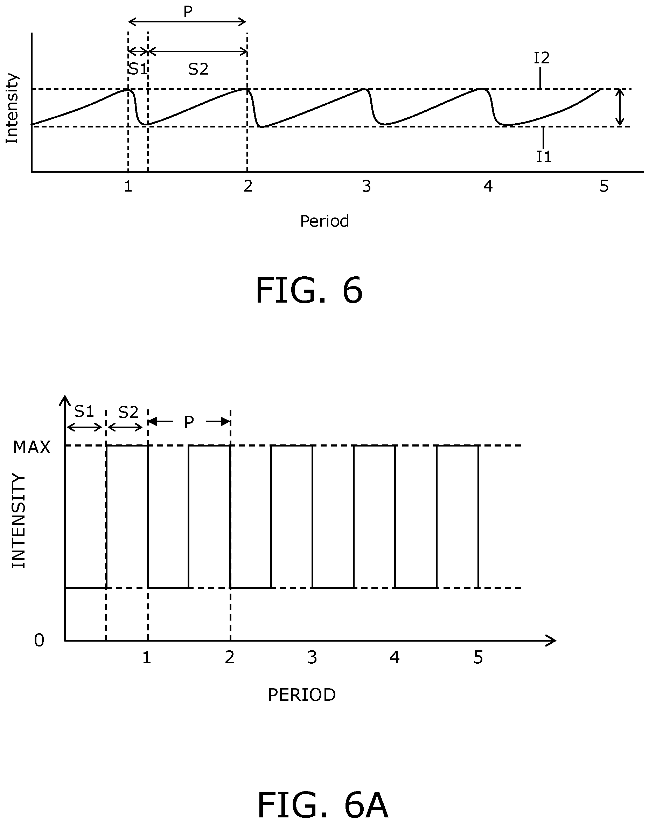

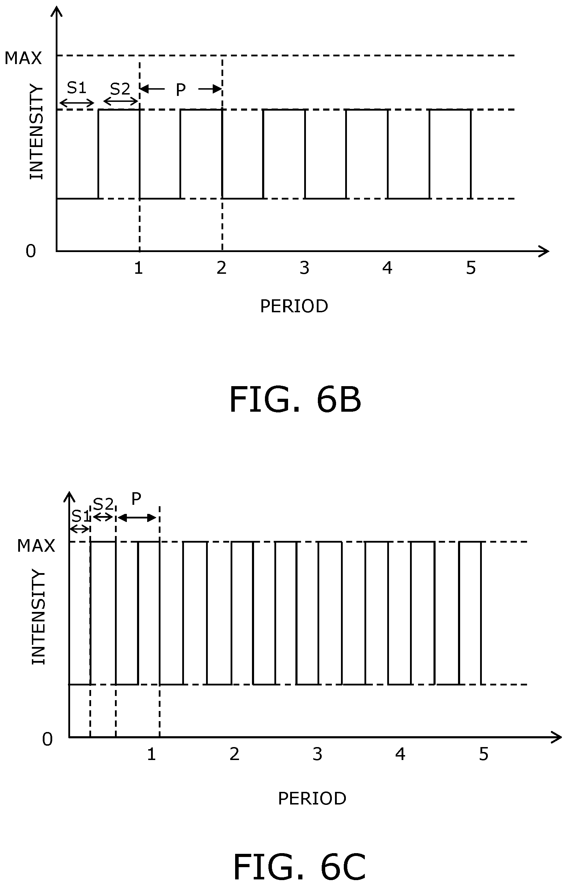

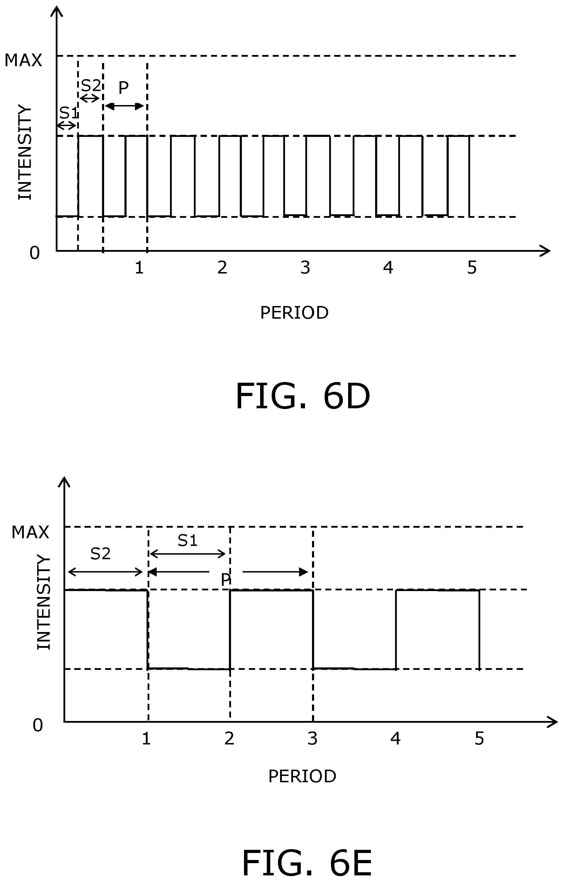

The controller 320 is configured to control an output of the at least one stimulating element 302. In some embodiments, the controller 320 is configured to control at least one of an intensity (amplitude), duration and frequency of an output of the at least one stimulating element 302. For example, in some embodiments the controller 320 is configured to cause the at least one stimulating element 302 to output in pulses (e.g., pulses of vibration, pressure application, rolling motion or tapping or other impact) as shown in Figure 6. Figure 6 depicts a pulse-like waveform. To achieve the pulse-like waveform shown in Figure 6 to be output from the at least one stimulating element 302, the controller 320 is configured to cause a continuous output of the at least one stimulating element 302 (either indefinitely or for a specified length of time) having periodic fluctuations in output intensity. For example, in a first segment S1 of each period P, an output intensity from the at least one stimulating element 302 may decrease from a second intensity level I2 to a first intensity level I1, before returning to the second intensity level I2 in a second segment S2 of the period P (or vice versa). In the output shown in Figure 6, the increase in output intensity from the first intensity level I1 to the second intensity level I2 is substantially linear. The decrease in output intensity from the second intensity level I2 to the first intensity level Ilis also substantially linear. However, the rate of change of output intensity from the second intensity level I2 to the first intensity level I1 is much greater than from the first intensity level I1 to the second intensity level I2. The difference in rates of change in segment S1 and segment S2 provides a pulsed effect in an output of the at least one stimulating element 302.

In the example waveform shown in Figure 6, the duration of the first segment S1 in each period P is approximately 15 % of the total duration of the period P. In alternative embodiments, the duration of the first segment S1 of each period P is between substantially 5 % and 25 % of the total duration of the period P. In some embodiments, the total duration of each period P is approximately 1 second (for example, a frequency of approximately 1 Hz). In alternative embodiments, the total duration of each period P is between substantially 0.25 seconds and 4 seconds (for example, a frequency of between substantially 4 Hz and 0.25 Hz). In some embodiments, the total duration of each period P is between substantially 0 second and 300 seconds (for example, a frequency of between substantially 300 Hz and 0 Hz). In some embodiments, the first intensity level I1 is substantially zero (e.g., substantially no output intensity from the at least one stimulating element 302). In some embodiments, each period P may comprise a third segment in which an output intensity level is substantially zero. In some embodiments, the third segment of the period P occurs between the first segment S1 and the second segment S2 as described with respect to Figure 6.

In a preferred embodiment, the vibrational stimulation is studied as a specialized pattern. The specialized pattern is purely mechanical and generates focused stimulation and cueing (like a metronome). Beneficially, the focused stimulation and cueing serve as therapy to Parkinson's disease and show a 32% efficacy as compared to 6% efficacy observed with the waveform. In a preferred embodiment, the wearable device 300 of the present disclosure integrates a combination of focused stimulation and cueing to help users with Parkinson's disease to regain their freedom of movement. Focused vibrational stimulation produces an effect similar to beta wave activity, as a result of activation of the neurons in synchronised oscillations. It will be appreciated that reduced beta wave activity is indicative of wakefulness while an increased beta wave activity is reflective of Parkinson's disease. Notably, sensory inputs, particularly from muscles regarding proprioception, are an important factor for initiation and execution of movements (as discussed by Hwang et al. 2016). The theory of active inference (as discussed by Friston et al. 2011) posits that a key step in modulating these inputs is aberrant in Parkinson's, and by increasing their messiness or noise by stimulating muscle receptors with focused vibration, their uncertainty can be increased and prompt the brain into adopting a more "ready-to-move" state (as discussed by Kording et al. 2006). Cueing is achieved by pulsing the vibrations. An appropriate cueing stimulation encourages regularity in elements of rhythmic movements like step length and limb coordination, thereby stabilizing and strengthening firing patterns of spinal neurons that generate locomotion. Moreover, cueing enables reinitiation of the spinal circuits in case of break down by subconsciously drawing attention away from other interrupting stimuli in the environment. Furthermore, cueing facilitates the execution of actions that directly precede and prepare for movements, which are impaired in Parkinson's disease. Beneficially, cueing provides a reduction in the freeze-of-gait episodes, leading to a smoother overall movement in people with Parkinson's disease.

Moreover, the intensity and the period of a stimulating pulse are parameters that can be set and modified by the user (such as my customising the wearable device 300). Once set, the pattern, with a minimum Intensity I1, a maximum intensity I2, and a period P, will be repeated as is, until further update by the user. It will be appreciated that the wearable device can be optimized as per the user preference for variable intensity of the pulses, such as I1 to I2, spanning across variable periods of time, such as P for a predefined duration to initiate a movement in the user. In an exemplary implementation, the device may be programmed at 100% intensity and 200ms ON/OFF period. Optionally, the said values may be adjusted by the user (using a mobile-based Application programme, as discussed in detail later). This allows the user to have full flexibility over the settings and adjust the values to what works best for each individual (as exemplified in Figures 6A, 6B, 6C, 6D and 6E). It will be appreciated that by controlling the intensity, i.e. decreasing it from 100% to 50%, the vibration is much softer and subtle.

In some embodiment, to achieve the focussed stimulation and cueing as shown in Figures 6A-6E to be output from the at least one stimulating element 302, the controller 320 is configured to cause generation of pulsed output of the at least one stimulating element 302 having periodic fluctuations in output intensity. The motor of the wearable device 300 is operable to run, for example, at 300 Hz and generate pulses. In some embodiments, the duration of the first segment S1 and the duration of the second segment S2 in each period P are substantially the same. In some embodiments, the total duration of each period P is approximately 1 second (for example, a frequency of approximately 1 Hz). In some embodiments, the total duration of each period P is between substantially 0.25 seconds and 4 seconds (for example, a frequency of between substantially 4 Hz and 0.25 Hz). In some embodiments, the total duration of each period P is between substantially 0 second and 300 seconds (for example, a frequency of between substantially 300 Hz and 0 Hz). In some embodiments, the first intensity level I1 is substantially zero (e.g., substantially no output intensity from the at least one stimulating element 302) and the second intensity level I2 is a positive number (e.g., maximum output intensity from the at least one stimulating element 302).

Figures 6A, 6B, 6C, 6D and 6E depict a pulsed stimulation at different duty cycles modified by the user, in accordance with various embodiments of the invention. The x-axis represents the intensity and the y-axis represents the time period. As shown in Figure 6A, at a 100% duty cycle, the motor of the wearable device 300 is either fully ON or fully OFF (at a voltage of for example 3V) as depicted by the intensity of the pulsed fluctuations at an I2 at maximum level (denoted as MAX) and I1 at 0, and the period between the segments S1 and S2 is 1 unit. As shown in Figure 6B, at a 50% duty cycle, the signal is being pulse-width modulation controlled and the intensity of the pulsed fluctuations is below a maximum level (denoted as MAX) while the period between the segments S1 and S2 is 1 unit. As shown in Figure 6C, intensity of the pulsed fluctuations is at a maximum level (denoted as MAX) and the period between the segments S1 and S2 is lesser than 1 unit, i.e. the pulses are generated at higher frequency. As shown in Figure 6D, intensity of the pulsed fluctuations is below a maximum level (denoted as MAX) and the period between the segments S1 and S2 is lesser than 1 unit, i.e. the pulses are generated at higher frequency. As shown in Figure 6E, intensity of the pulsed fluctuations is below a maximum level (denoted as MAX) and the period between the segments S1 and S2 is 2 units, i.e. the pulses are generated at a low frequency.

In some embodiments, the controller 320 is or comprises a printed circuit board (PCB). In some embodiments, a power source such as a battery is mounted on the PCB. Alternatively, the battery may be provided on one or more of the coils of wire in the charging portion 216, with power being provided by the unit 250.

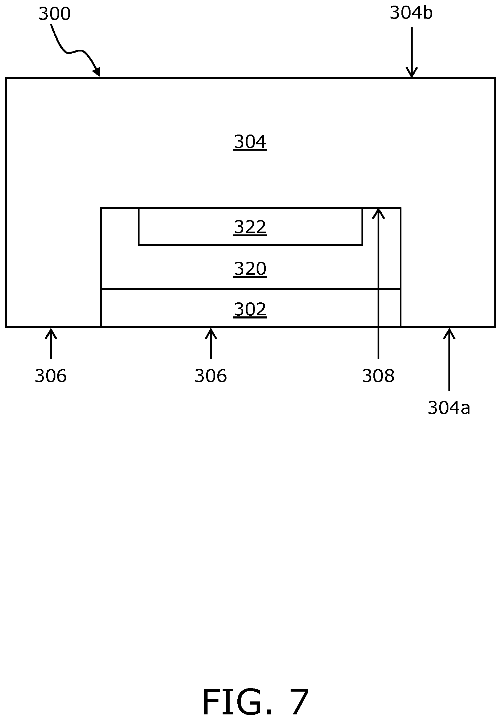

The controller 320 comprises a user input 322 (for example, a switch or touch sensor) mounted on the PCB. The user input 322 is configured to receive commands from a user to provide instructions to the controller 320 to control an output of the at least one stimulating element 302. In some embodiments, the user input 322 is configured to be actuated via touch of or application of pressure to an outer surface of the wearable device 304. In some embodiments, the user input 322, in the form of a touch or pressure application site, is located or disposed within the wearable device 300 substantially opposite the surface 306 configured to contact skin of the user. This may improve ease of access to the user input 322 when the wearable device 300 is being worn or is adhered to the user. In some embodiments, an outer surface of the wearable device 300 substantially opposite the surface 306 is actuated to actuate the user input 322, for example the second end 304b of the dissipating portion 304 (or a capping layer 310 covering an open second end 304b of the dissipating portion 304). In some embodiments, the user input 322 is configured to be actuated via touch of or application of pressure to a button provided on the outer surface of the wearable device 304. The button is located or disposed in the middle of the wearable device 300 substantially opposite the surface 306 configured to contact skin of the user. Such button enables touch or pressure application to be detected by the user input 322 in order to provide instructions to the controller 320. In alternative embodiments, for example a dissipating portion 304 having open first and second ends 304a, 304b and no capping layer, the user input 322 is exposed and is configured to be actuated directly via touch of or application of pressure to the user input 322. As shown in Figure 7, in some embodiments the user input 322 is located adjacent the second end 304b of the dissipating portion 304, within the recess or aperture 308 of the dissipating portion 304. Such arrangements enable touch or light pressure application of the second end 304b (or the capping layer 310) to be detected by the user input 322 in order to provide instructions to the controller 320. In some embodiments, the controller 320 is configured to receive and determine between a plurality of different commands received via the user input 322. For example, the different commands may comprise different durations of touch or pressure application, or may comprise a different number of touches or pressure applications within a pre-determined time period. Each of the different commands may result in the controller 320 causing the at least one stimulating element 302 to provide a different output (for example, a different length, intensity, frequency of output, a different output waveform etc.) In some embodiments, the controller 320 is configured to receive and determine between three or more different commands received via the user input 322. In an example, 2 continuous touches or pressure application (taps) result in the controller 320 causing the at least one stimulating element 302 to turn on the Bluetooth^{®}. In an example, 1 long continuous touch or pressure application (tap) for an extended period of time, such as 2 seconds, result in the controller 320 causing the at least one stimulating element 302 to provide instructions to turn on or off the wearable device 300.

In some embodiments, the wearable device 300 comprises a light emitting diode (LED) layer. The LED layer is located or disposed within the wearable device 300 substantially opposite the surface 306 configured to contact skin of the user. The LED layer may be located or disposed on the printed circuit board (PCB) and is visible through a transparent silicon surrounding the touch or pressure application site or the button. In some embodiment, the LED indicates different levels of charging (of the battery) in the wearable device 300. In an example, different intensities of a light by the LED layer, such as blue light, is indicative of different levels of charging of the wearable device 300. For example, a bright blue light symbolizes a substantially charged battery while a faint blue light represents a substantially drained battery. In another example, different colours of light emitted by the LED layer may be indicative of different levels of charging of the wearable device 300. For example, a green light symbolizes a fully-charged battery, a blue light represents a partially-charged battery, and a red light suggests the recharging the battery. In some embodiments, the LED layer indicates medication alerts. This may improve ease of access to the user input 322 when the wearable device 300 is being worn or is adhered to the user.

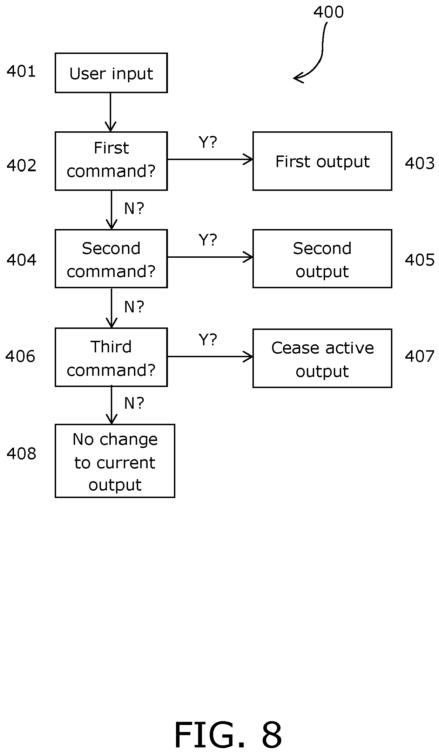

A schematic illustration of a control scheme 400 provided by the controller 320 in response to instructions provided using user input 322 is shown in Figure 8.

At step 401, the user actuates the user input 322 to provide instructions to the controller 320.

At step 402, the controller 320 determines whether or not the actuation of the user input 322 corresponds to a first command. In some embodiments, the first command comprises a single touch or pressure application (for example, with a substantially immediate release of the touch or pressure application after actuating the user input 322). If the controller 320 determines that the first command has been received at step 402, the controller 320 instructs the at least one stimulating element 302 to provide a first output at step 403. In some embodiments, the first output of the at least one stimulating element 302 comprises up to two minutes of pulsed output. In alternative embodiments, the first output of the at least one stimulating element 302 comprises between one and five minutes of pulsed output. If at step 402 the controller 320 does not determine that the first command has been received, the controller 320 proceeds to step 404.

At step 404, the controller 320 determines whether or not the actuation of the user input 322 corresponds to a second command. In some embodiments, the second command is different to the first command. In some embodiments, the second command comprises a double touch or pressure application (for example, each touch having a substantially immediate release of the touch or pressure application after actuating the user input 322, each of the two touches being received with a pre-determined period such as up to two seconds). In alternative embodiments, the second command comprises a press-and-hold or touch-and-hold (for example, continuing touch or pressure application for a pre-determined time period such as one second). If the controller 320 determines that the second command has been received at step 404, the controller 320 instructs the at least one stimulating element 302 to provide a second output at step 405. In some embodiments, the second output of the at least one stimulating element 402 comprises continuous pulsed output until the controller 320 receives a command instructing it to cease or prevent output of the at least one stimulating element 302, with the pulse-like waveform of the second output being substantially similar to the pulse-like waveform of the first output. In other embodiments, the pulse-like waveforms of the first output and the second output are different. In such embodiments, the duration of the second output comprises continuous pulsed output until the controller 320 receives a command instructing it to cease or prevent output of the at least one stimulating element 302, or comprises pulsed output for a pre-determined period of time. In some embodiments, the pre-determined period of time of the second output is between one and five minutes. The second output may be continuous e.g. for up to approximately 30 minutes from turning on. If at step 404 the controller 320 does not determine that the first command has been received, the controller 320 proceeds to step 406.

At step 406, the controller 320 determines whether or not the actuation of the user input 322 corresponds to a third command. In some embodiments, the third command is different to the first and second commands. In some embodiments, the third command comprises a double or triple touch or pressure application (for example, each touch having a substantially immediate release of the touch or pressure application after actuating the user input 322, each of the two or three touches being received with a pre-determined period such as up to two seconds). In alternative embodiments, the second command comprises a press-and-hold or touch-and-hold (for example, continuing touch or pressure application for at least a pre-determined time period such as one second or two seconds). If the controller 320 determines that the third command has been received at step 406, the controller 320 instructs the at least one stimulating element 302 to cease output, if the at least one stimulating element 302 is currently providing an output. If the at least one stimulating element 302 is not currently providing an output when the third command is received, the controller 320 instructs the at least one stimulating element to remain providing zero output. If at step 406 the controller 320 does not determine that the third command has been received, the controller 320 proceeds to step 408. At step 408, the controller 320 determines that it does not recognise the user input, and provides no instruction to the at least one stimulating element 302. In that case, there is no change to the current output of the at least one stimulating element 302. If the at least one stimulating element 302 is providing an output at step 408, it continues to provide that output, and if the at least one stimulating element 302 is not providing an output at step 408, it continues to not provide an output.

Applying a mechanical stimulus (such as vibration, pressure change, rolling motion, tap or other impact) to sufferers of neurological conditions or diseases such as Parkinson's disease may reduce or eliminate freeze of gait, tremors, stiffness or slowness. The application of a mechanical stimulus may reduce or suppress beta waves which may in turn alleviate physical symptoms of such neurological diseases such as Parkinson's disease. However, applying a mechanical stimulus which is too strong (too high in intensity), whilst being effective in targeting physical symptoms, may cause irritation and discomfort to patients. Conversely, applying a mechanical stimulus which is too weak (too low in intensity) simply does not effectively target physical symptoms. By applying a pulse-like mechanical stimulus (for example, fluctuating between different intensity levels of the mechanical stimulus such as a first intensity level and a second intensity level higher or lower than the first intensity level), effective alleviation or removal of physical symptoms can be obtained without causing discomfort or irritation. An additional advantage of applying a pulse-like mechanical stimulus is that the frequency and intensity fluctuations of the mechanical stimulus can be controlled in order to provide cue signs to an individual (in effect, the pulse-like mechanical stimulus may also act as a metronome). By providing a regular, rhythmic output, the pulse-like mechanical stimulus can also modulate sensory dysfunction by enabling individuals to focus on the regular rhythm and coordinate timings of their own movement to the pulse-like mechanical stimulus. In doing so, freeze of gait, tremors, stiffness or slowness may be additionally or further reduced. In some embodiments, the wearable device 100, 200, 300, is placed on the sternum of a user to apply a mechanical stimulus to the user to alleviate physical symptoms of a neurological condition such as freeze of gait, stiffness, slowness or tremors. In alternative embodiments, the wearable device 100, 200, 300 is placed on another area of the body of a user, for example, on a back of the hand, wrist, foot, or any other suitable position on the body. The surface 106, 206, 306 configured to deliver a mechanical stimulus to the user is placed in contact (direct, or indirect for example via an adhesive) with skin of the user. Application on the sternum provides easy access for a user to control the wearable device 100, 200, 300, for example using a user input 322 connected to a controller 320 (or simply to turn the wearable device 100, 200, 300 on). Beneficially, the application on the sternum provides a larger surface area for the functioning of the wearable device 100, 200, 300 and easy access (i.e. visually) to and/or interaction with the device by family or a carer of the user, for example, pressing the button, putting it ON or OFF, taking it off, and so on. It will be appreciated that other parts of the body, which are accessible to the user as well as visible and/or accessible to the family or carer thereof, may be selected for the wearable device 100, 200, 300 to be placed thereon. Such other body part may include, but are not limited to, ankle, leg, neck, arm, shoulder, wrist and temple.

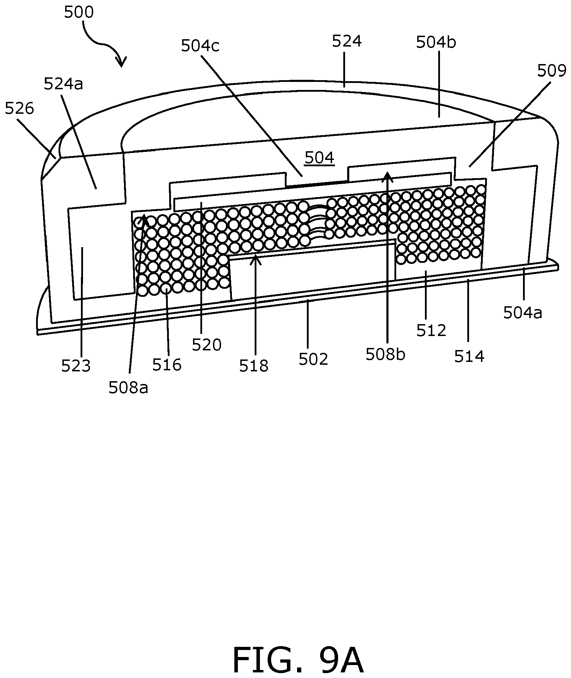

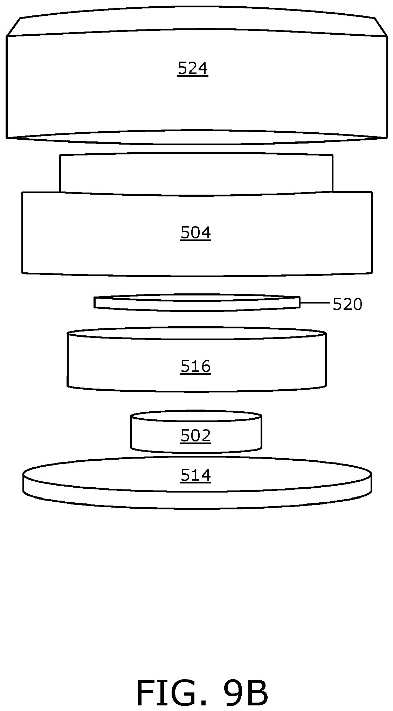

Figures 9A and 9B show an embodiment of a wearable device 500 in accordance with the invention. The wearable device 500 comprises substantially similar features to those described with respect to the wearable devices 100, 200, 300 above (like or corresponding reference numerals are used where relevant). Figure 9B shows an exploded view of the embodiment shown in Figure 9A. In the embodiment shown in Figure 9A, a dissipating portion 504 is or comprises a substantially cylindrical shape having an open first end 504a and a closed second end 504b. The dissipating portion 504 comprises a recess 508. The recess 508 is configured to receive at least one stimulating element 502, a charging portion 516, and a controller 520. In the embodiment shown, the recess 508 comprises a first recess portion 508a and a second recess portion 508b. The second recess portion 508b is smaller than the first recess portion 508a (for example, the second recess portion 508b has a smaller depth (in the proximal-distal direction) and diameter (transverse to the proximal-distal direction) than the first recess portion 508a). The second recess portion 508b is disposed or located further from the first open end 504a than the first recess portion 508a (for example, the second recess portion 508b is disposed at a greater depth within the recess 508 than the first recess portion 508a). The second recess portion 508b is disposed more towards the distal end of the device 500 than the first recess portion 508a. A shoulder 509 is formed between the first recess portion 508a and the second recess portion 508b due to the difference in size between the first recess portion 508a and the second recess portion 508b. The shoulder 509 effectively forms a secondary mouth for the second recess portion 508b. In the embodiment shown, the controller 520 (for example, being or comprising a printed circuit board (PCB)) is received within the second recess portion 508b. The controller 520 is configured to be substantially flush with the secondary mouth of the second recess portion 508b (for example, substantially flush with the shoulder 509).

The charging portion 516 is disposed within the first recess portion 508a. The shoulder 509 prevents the charging portion 516 from entering the second recess portion 508b. The shoulder 509 forms at least part of a surface within the recess 508 on which the charging portion 516 is disposed to securely receive the charging portion 516 within the recess 508. In the embodiment shown, the controller 520 is disposed adjacent the charging portion 516. This allows the controller 520 to be easily connectable to the charging portion 516. In some embodiments, the charging portion 516 is connected to a power source such as a battery (for example, connected to the controller 520 to power the controller 520, or mounted on a PCB forming part of the controller 520). In the embodiment shown, the controller 520 also forms part of the surface (together with the shoulder 509) configured to receive the charging portion 516. In the embodiment shown, the charging portion 516 does not extend through a full depth of the first recess portion 508a. Rather, the charging portion 516 is disposed so that it is set back from a mouth of the recess 508 (for example, set back from the first end 504a of the dissipating portion 504). This allows an adhesive 512 (such as a layer of adhesive) to be disposed on the surface 506 configured to deliver a mechanical stimulus to the user.

In the embodiment shown, the charging portion 516 comprises a recess 518 configured to at least partially receive the at least one stimulating element 502. This enables the at least one stimulating element 502 to be securely retained, relative to the dissipating portion 504, within the recess 508.

In the embodiment shown, the charging portion 516 also acts to indirectly mechanically connect the dissipating portion 504 to the at least one stimulating element 502. As such, a mechanical stimulus provided by the at least one stimulating element 502 can be transmitted through the charging portion 516 to the dissipating portion 504. The dissipating portion 504, forming at least a part of the surface 506, is therefore configured to increase an effective area of the mechanical stimulus provided by the at least one stimulating element 502 (for example, the radius of the dissipating portion 504 is larger than a radius of the at least one stimulating element 502, enabling the dissipating portion 504 to apply the mechanical stimulus over a larger surface area than that of the at least one stimulating element 502). In other embodiments having a different internal arrangement of components, the dissipating portion 504 is in direct contact with the at least one stimulating element 502 to increase an effective area of the mechanical stimulus.

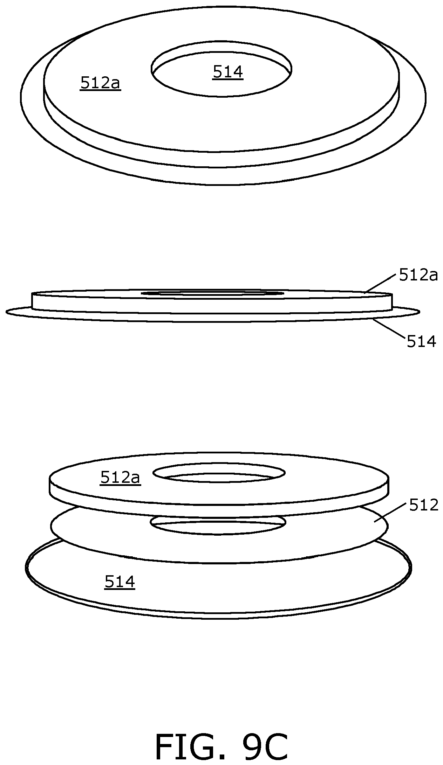

In the embodiment shown, the at least one stimulating element 502 extends beyond a depth of the recess 518 in which it is received. In the embodiment shown, the at least one stimulating element 502 is substantially flush with the surface 506 (for example, substantially flush with a first end 504a of the dissipating portion 504). The arrangement of the charging portion 516 (set back from the surface 506), the at least one stimulating element 502 and the dissipating portion 504 in the embodiment shown provides a ring-shaped area adjacent the surface 506 (between the dissipating portion 504 and the at least one stimulating element 502). The ring-shaped area is configured to receive an adhesive 512 which in turn enables the wearable device 500 to be adhered to skin of the user. In the embodiment shown, the adhesive 512 is substantially level with the first end 504a of the dissipating portion 504 and the at least one stimulating element 502 forming the surface 506. In alternative embodiments, the adhesive 512 may be applied onto the surface 506 itself. In the embodiment shown, the adhesive 512 also aids in sealing the wearable device and retaining the charging portion 516, the at least one stimulating element 502 and the controller 520 within the recess 508 of the dissipating portion 504. A removable and replaceable cover 514 is provided to cover the adhesive 512 when the wearable device 500 is not adhered to skin of the user. In alternative embodiments, the adhesive 512 is disposed on a soft layer of material 512a (being or comprising, for example, urethane, silicone, rubber or another flexible material) configured to be received within the ring-shaped area to form a substantially constant, level surface 506 (for example, level or substantially flush with the first end 504a of the dissipating portion 504). This is shown in Figure 9C. The soft layer of material 512a may have or comprise a shape substantially similar to the ring-shaped area. The adhesive 512, soft layer 512a and a cover 514 may be provided as a single entity or insert configured to be applied to or inserted into the recess 508 of the wearable device 500 before application to skin of the user. The insert may be applied to the wearable device 500 by inserting the soft layer 512a into the ring-shaped area in the recess 508. The cover 514 may then be removed or peeled back to expose the adhesive 512 in order to adhere the wearable device 500 to skin of the user. The cover 514 may prevent exposure of the adhesive 512 prior to adhering the surface 506 to skin of the user. A plurality of inserts may be disposed in the reservoir 260 of the docking station 250 (as described above) and may be retrieved individually when required.

In preferred embodiments, the adhesive 512 is disposed in a hook-and-loop fastener as shown in Figure 4J, for example a Velcro^{™}. The respective backs of the hook layer and the loop layer comprise adhesives. Adhesives on the hook layer and the loop layer are of different grades and strength. A medical grade adhesive is provided at the back of the loop layer that faces the skin of the user for attachment thereon, and a strong adhesive is provided at the back of the hook layer that attaches with the wearable device 500. The wearable device 500 can be removed for charging or when not required for use, with the loop layer with the medical-grade adhesive remaining attached on the skin, while the hook layer with the stronger adhesive remaining attached on the wearable device 500. This enables the wearable device 500 to be used continuously, i.e. 24 hours a day, or intermittently, i.e. removed for charging. Eventually when the adhesive stops to show its effect on the skin, the adhesive can be replaced with a new layer of adhesive. Moreover, the adhesive corresponding to the hook layer is strong enough to last several months if not years. However, the hook layer can also be detached from the wearable device 500 in case the hook layer wears off.

In the embodiment shown, the dissipating portion 504 comprises a protrusion 504c extending from the second end 504b into the recess 508 (in particular, into the second recess portion 508b). The protrusion 504c is configured to interact with a user input (not shown, but similar user input 322 described above with respect to the wearable device 300). In the embodiment shown, therefore, the user input is actuated by actuating the second end 504b of the dissipating portion 504 to bring the protrusion 504c into contact with the user input, or to apply detectable pressure to the user input using the protrusion 504c. It will be appreciated that a similar effect could be achieved if the second end 504b was open, but a capping layer comprising a protrusion was used instead. In alternative embodiments, the second end 504b of the dissipating portion 504 is open, and the user input is left exposed to be actuated by the user directly. As can be seen, the user input is disposed substantially opposite the first end 504a of the dissipating portion 504 (for example, substantially opposite the surface 506) in order to improve ease of access to the user input whilst the wearable device 400 is adhered to skin of the user. The wearable device 500 may not need to be removed from skin of the user in order for the user to access or actuate the user input to provide instructions to the controller 520 to control an output of the at least one stimulating element 502.

In the embodiment shown, the wearable device 500 also comprises a housing 524. The housing 524 is configured to partially surround the dissipating portion 504. The first end 504a and the second 504b of the dissipating portion 504 are not fully covered or surrounded by the housing 524, so as not to interfere with proper functioning of the wearable device 500. In the embodiment shown, the housing 524 comprises a circular opening or aperture at each end, each of the respective ends of the housing 524 located adjacent one of the first end 504a and the second end 504a of the dissipating portion. In some embodiments, the housing 524 comprises a plastic material (for example, polypropylene or polycarbonate) configured to protect the dissipating portion 504 from impact, scratches or other degradation which could affect performance of the dissipating portion 504. In the embodiment shown, an internal surface of the housing 524 comprises a shoulder 524a configured to interact with a shoulder 523 on an external surface of the dissipating portion 504. The shoulders 523, 524a are configured to be mating surfaces when the housing 524 is correctly located on the dissipating portion 504. An adhesive may be used to connect the shoulders 523, 524a to secure the housing 524 to the dissipating portion 504. In alternative embodiments in which a capping layer is used with an open second end 404b of the dissipating portion, the capping layer is configured to contact and/or join to the second end 504b of the dissipating portion 504. In such embodiments, a shoulder on (for example, on a perimeter of) the capping layer may interact with the shoulder 524a of the housing 524. The capping layer may substantially fill the circular aperture of the housing 524 adjacent the second end 504b of the dissipating portion 504. A user input disposed adjacent the second end 504b may be actuated by the application of touch or pressure to the capping layer. In further alternative embodiments, no capping layer is provided, and the housing 524 is disposed directly onto an open second end 504b of the dissipating portion 504 (for example, a solid surface of the second end 504b rather than the aperture 508). The flat, open second end 504b of the substantially cylindrical dissipating portion 504 is configured to act as a shoulder to interact with the shoulder 524a of the housing 524. A circular aperture of the housing 524 adjacent the second end 504b enables direct access for the user to actuate a user input disposed adjacent the second end 504b (that is, substantially opposite the first end 504a, or substantially opposite the surface 506). It will be appreciated that a housing such as the housing 524 could be incorporated into any of the wearable devices 100, 200, 300 described above.

In some embodiments, the housing 524 comprises a metal casing, such as for example aluminium casing, preferably anodized aluminium, configured to protect the dissipating portion 504 from impact, scratches or other degradation which could affect performance of the dissipating portion 504. Beneficially, the aluminium casing enables reducing noise by serving as an excellent sound absorber to sound produced within the wearable device (i.e. noise produced by motor, controller or battery). Additionally, beneficially, aluminium casing is lightweight, extremely strong, naturally protects electronics, and comes with beautiful finishes thereby increasing the visual appeal of the wearable device 100. Furthermore, aluminium is 100% recyclable and non-toxic material, thereby highly environmentally friendly,

In some embodiments, the housing 524 comprises a step layer 526. The step layer 526 is produced by creating a groove or a furrow (similar to a chamfer or a bevel in carpentry) in the housing 524. The step layer 526 is typically created at an angle ranging between 20° and 75°, preferably 45°, in the outer surface of the housing 524. The step layer 526 comprises a continuous lining that blends in the housing 524 without producing sharp edges both for safety of the user as well as preventing damage to the edges of the wearable device 500. The ergonomic design of the wearable device 500 enable comfortable handling and other physical working conditions, such as a firm grip, suitable for a patient with restricted movements, such as patients suffering from Parkinson's disease. In this regard, the cylindrical design of the wearable design 500 along with the step layer 526 prevents the risk of knocking off and/or dropping the wearable device 500 accidentally while using or taking off the wearable device 500. Beneficially, the step layer 526 enables comfortable grab of the wearable device 500, both while placing it on the user's skin and taking it off. Additionally, beneficially, the step layer 526 allows water or sweat to run off of the wearable device 500. This enables the wearable device 500 to be attached to the user's skin for a longer period of time. In some embodiments, the wearable device 500 can be used 24 hours a day to assist patients seeking vibrational stimulation for starting their day (for example, waking up) and through the day.

The features and interaction of the various components of the wearable device 500 allow for a small, compact device without any loss in performance or functionality, enabling easy operation by a user both whilst adhered and not adhered to skin of the user. It will be appreciated that this is also true if the wearable device 500 is not adhered to skin of the user, but instead incorporated in a wearable item and retained in contact with skin of the user via the wearable item (as described above).

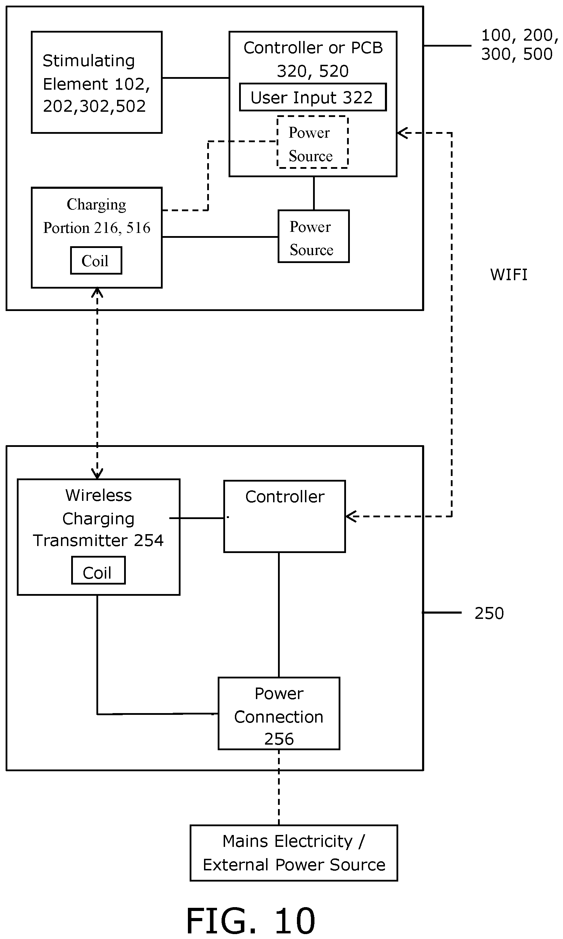

Figure 10 shows a schematic illustration of an embodiment of a wearable device 100, 200, 300, 500 and a docking station or unit 250 in accordance with the invention. The schematic illustration depicts how various components of the wearable device 100, 200, 300, 500 and the unit 250 interact or communicate with one another in order to operate as intended. As shown in Figure 10, the power connection 256 of the unit 250 is configured to be connected to mains electricity or another external power source. The power connection 256 is in communication (e.g., electrical communication) with the wireless charging transmitter 254. In the embodiment shown, the power connection 256 is also in communication with a controller of the unit 250. In some embodiments, the controller of the unit 250 is configured to control power delivery from the power connection 256 to the wireless charging transmitter 254, for example in order to control efficiency of wireless power transfer from the wireless charging transmitter 254 to the charging portion 216, 516 of the wearable device 100, 200, 300, 500. The power connection 256 is also configured to provide power to the controller of the unit 250. The wireless charging transmitter 254 is configured to wirelessly transmit power to the charging portion 216, 516, as indicated by the dashed line between the charging portion 216, 516 and the wireless charging transmitter 254. In some embodiments, the power connection 256 may connect the wireless charging transmitter 254 more directly to the external power source (for example, not via a controller of the unit 250). In some embodiments, the controller of the unit 250 is configured to communicate (for example, via a wired connection, or via a wireless connection such as WIFI, as indicated in Figure 10) with the controller 320, 520 of the wearable device 100, 200, 300, 500. For example, the controller 320, 520 of the wearable device 100, 200, 300, 500 may determine when a power source of the wearable device 100, 200, 300, 500 is fully charged, and may communicate with the controller of the unit 250 to instruct the wireless charging transmitter 254 to cease wirelessly transmitting power to the charging portion 216, 516 of the wearable device 100, 200, 300, 500.

The charging portion 216, 516 of the wearable device 100, 200, 300, 500 is in communication with and configured to deliver electric power to the power source (for example, battery) of the wearable device 100, 200, 300, 500. In some embodiments, the power source is located separately from the controller or PCB 320, 520, as depicted by the solid outline of the power source in Figure 10 (and associated solid lines indicating a connection between the power source and the controller 320, 520 and the charging portion 216, 516). In alternative embodiments, the power source is disposed or mounted on (for example, is integrated into or is part of) the controller 320, 520 of the wearable device 100, 200, 300, 500, as depicted by the dashed outline of the power source in Figure 10 (and associated dashed line connecting the power source to the charging portion 216, 516). The power source is configured to provide power to the controller or PCB 320, 520 and the at least one stimulating element 102, 202, 302, 502 of the wearable device 100, 200, 300, 500. The controller 320, 520 is configured to communicate with the stimulating element 102, 202, 302, 502 in order to provide instructions to control an output of the stimulating element 102, 202, 302, 502 whilst it is operational. In the embodiment shown, the controller 320, 520 is in communication with a user input 322 (which may be a part of, for example mounted on, the controller 320, 520). The user input 322 may be a depressable switch or a touch sensor. The user input 322 is configured to be actuated by a user in order to provide instructions from the user to the controller 320, 520 in order to control an output of the stimulating element 102, 202, 302, 502, for example as discussed above.

In some embodiments, a wearable device 100, 200, 300, 500 comprises additional components to increase or improve functionality. For example, as the wearable device 100, 200, 300, 500 may be used to alleviate or eliminate physical symptoms associated with various neurological conditions which often require medication, the wearable device 100, 200, 300 500 may comprise a medication reminder. The medication reminder may take the form of an alert such as an audible or visible alert emitted from the wearable device. Alternatively, the medication reminder may comprise a further mechanical stimulus from the at least one stimulating element 102, 202, 302, 502. The medication reminder may be configured to be active at particular times, corresponding to specific times of day at which medication should be taken, or configured to be active after a pre-determined period of time after an output of the at least one stimulating element 102, 202, 302, 502. The specific times of day or pre-determined period of time may be programmed into the controller 320, 520. Additionally or alternatively, the wearable device 100, 200, 300, 500 may further comprise a fall detection system. The fall detection system may comprise one or more accelerometers configured measure movement of the user. Readings from the one or more accelerometers may be analysed (for example, by the controller 320, 520) to identify if the user has fallen. The wearable device 100, 200, 300, 500 may further comprise an alert system (for example, an audible, visible or tactile alert) configured to indicate that the user has fallen.

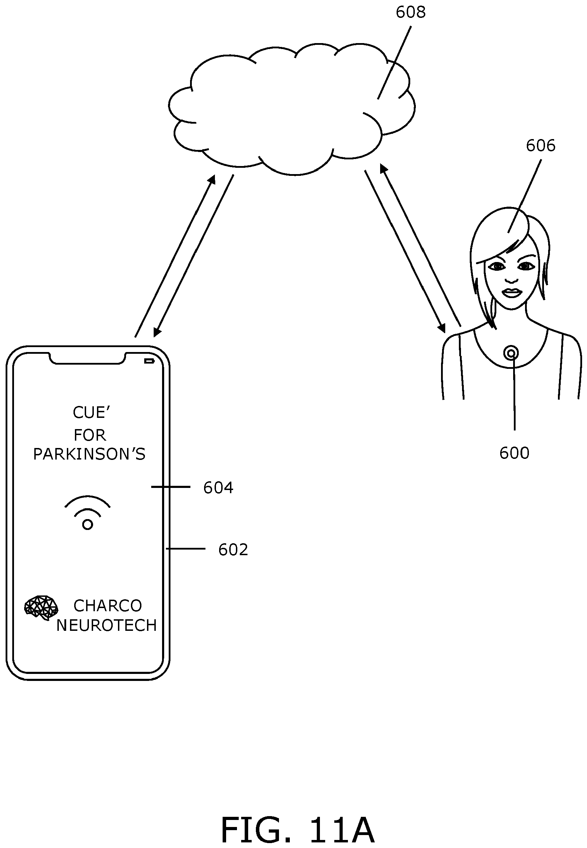

In some embodiments, the wearable device 600 is configured for tracking by an application software, when in operation, as shown in Figure 11A. The wearable device 600 has a to-use application software that is integrated with an external device 602 (referred to as "*device*" hereafter). Such to-use application software integrated in at least one compatible device 602 is referred to as a device integration application. A compatible device 602 is configured to run the device integration application thereon. The device 602 includes, but is not limited to, a mobile, a laptop computer, a tablet computer, a desktop computer, a palmtop computer and a smart watch. The term "*device integration application*" refers to a software program for execution by the device 602 by employing the processor of said device 602. Notably, the device integration application comprises a set of predefined functions that are programmed to provide instructions to hardware and/or software elements of the device 602. Furthermore, the device integration application is configured to provide a user interface on the display 604 of the device 602, to allow the user to perform specific associated tasks. In an example, the device integration application is an application programming interface. In an embodiment, the device integration application is affiliated to an organisation. Therefore, the device integration application functions in accordance with pre-programmed guidelines provided by the organisation. The device integration application is configured to function in accordance with the pre-programmed guidelines upon installation thereof.

In some embodiments, the device integration application enables the user 606 to apply touch or light pressure as a cue to access the user input 322 when the wearable device 300 is being worn or is adhered to the user (i.e. when in operation). The touch or light pressure application by the user 606 on the user input 322 is configured to provide instructions to the controller 320 to instruct the at least one stimulating element 302 to provide an output based on the received user input 322. In some embodiment, the device integration application enables the user 606 to instruct the device 602 when to remind them to take prescribed medicines. In this case, the device integration application includes prescription information related to, for example, names of medicines, doses of medicines, time of taking medicine, and so on. Moreover, the device integration application allows configuring the wearable device 600 based on user's requirement (in other words, is tailored to meet specific requirements). In such embodiment, the device integration application enables producing an identifiable alert in addition to a stimulation alert. Beneficially, the identifiable alert comprises an alert pattern, such as visual, audible, or a combination thereof, for the family or carer of the user 606 to help an incapacitated user 606. In some embodiments, the device integration application allows scheduling appointments with the doctor and sending an advanced notification (or reminder) therefor. It will be appreciated that the advanced notifications may be generated based on a predefined period, for example, 2 days, 1 day, 6 hours, and 2 hours before the due appointment.

In some embodiments, the device integration application enables tracking and recording the regular use of the wearable device 600, symptoms, variations and progression of the symptoms, and the efficacy of the wearable device 600 as well as the therapies (including medicines, meditation, etc.) on the symptoms. In some embodiments, the device integration application is configured to collect and analyse the data recorded by the user 606 and use it to measure and provide improvement as graphs. In some embodiments, the device integration application reports (for example sends via email or another device integration application) day-to-day analysis of the wearable device 600 to the user's doctor or physician via a communication network 608. Additionally or alternatively, the device integration application triggers an automatic alert when the readings go beyond a predefined threshold. In an alternate embodiment, the wearable device 600 is operable to be used without any Bluetooth^{®} connection. The wearable device 600 comprises a first command, i.e. touch or pressure application once for 5 minutes stimulation, and a second command for continuous stimulation. In such embodiment, the wearable device 600, through the device integration application, is configured to provide medication alert, as well as manage the alert system, vibration amplitude, duty cycle, vibration wave form, battery status and on/off of the wearable device 600. In some embodiments, the device integration application is used to trigger the wearable device 600 from a remote location, for example by the user's doctor, carer, or a family member authorised to access the wearable device in an emergency situation. It will be appreciated that the wearable device 600 is a stand-alone device and requires no new software or hardware for its functioning.

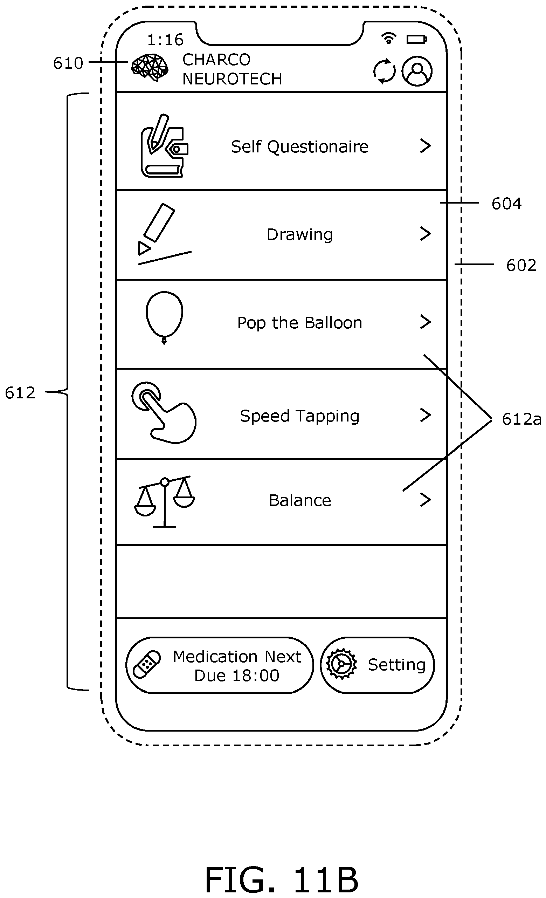

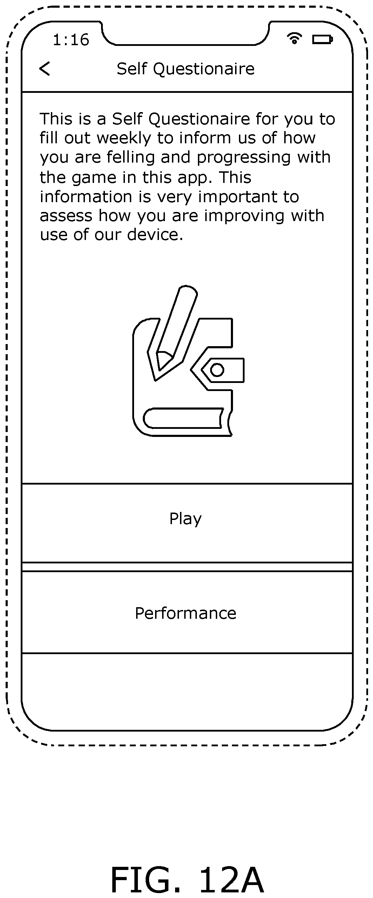

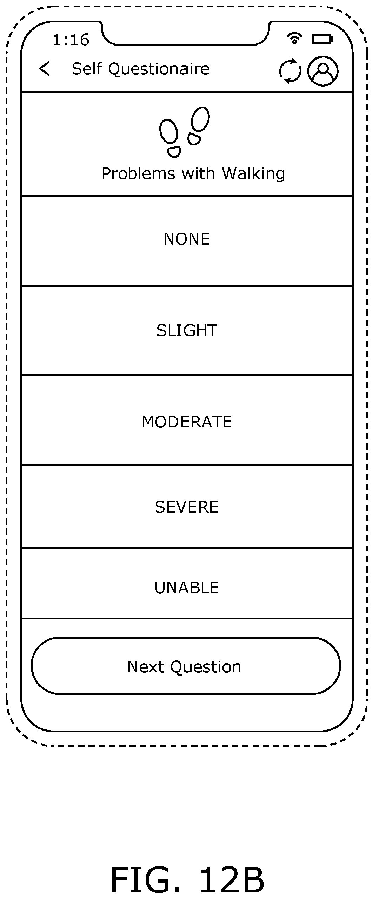

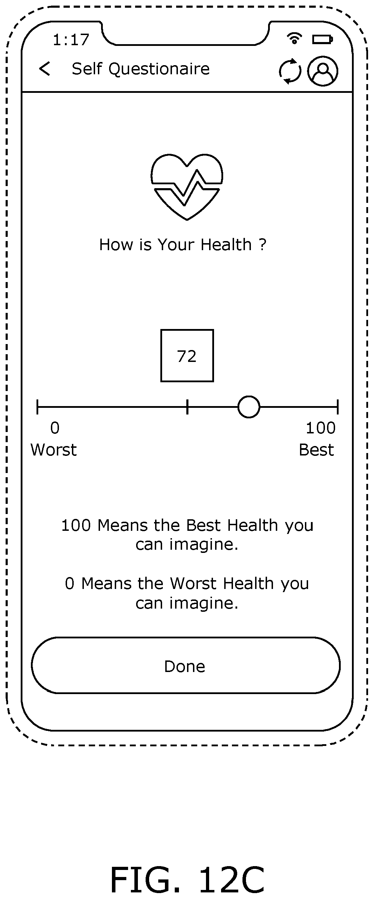

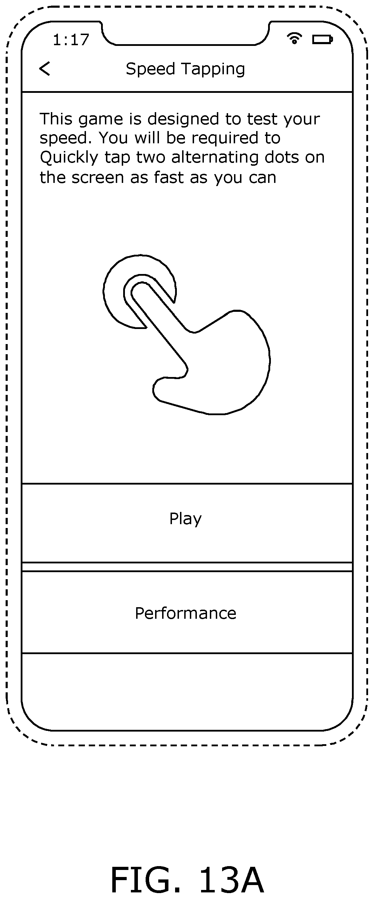

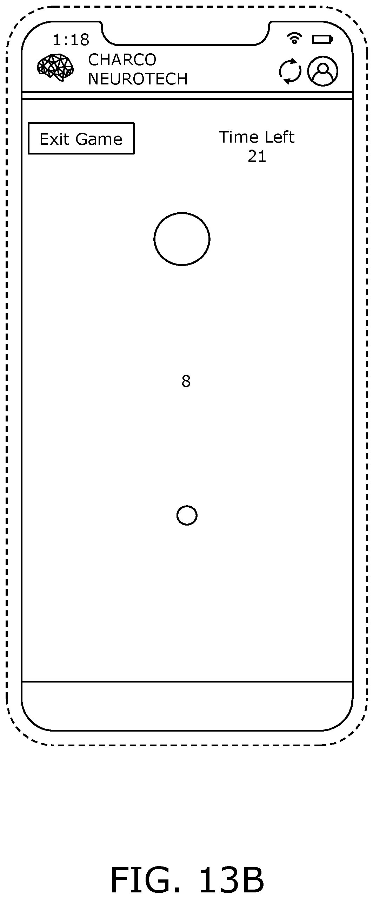

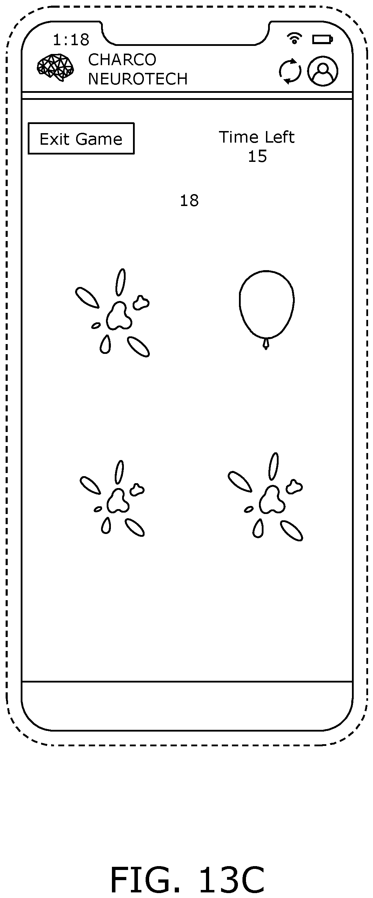

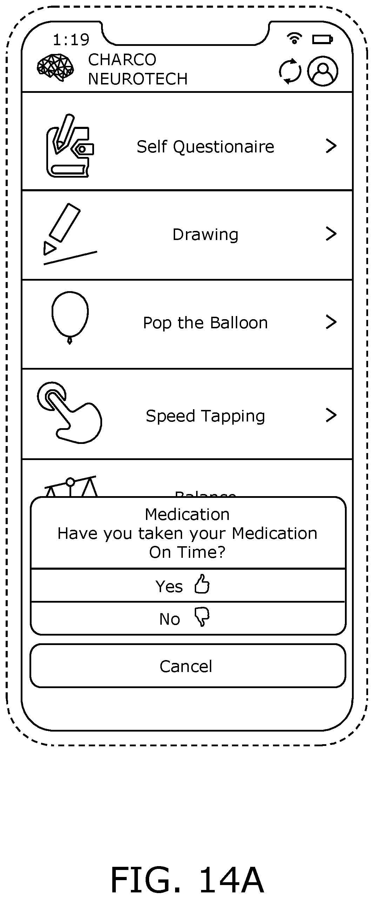

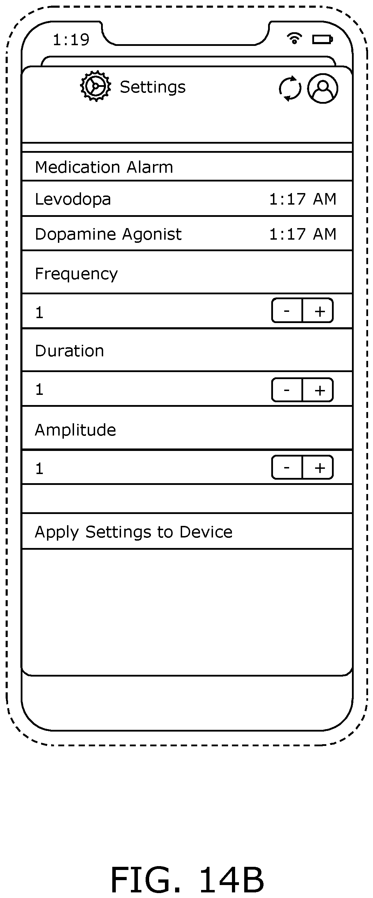

Figure 11B is an exemplary implementation of a successful installation of the device integration application on a device 604, such as a smart phone. As shown, a logo or icon 610 of the device integration application is visible on the smart phone display 604. The device integration application comprises a main menu 612 with a plurality of options (as shown in detail in Figures 12A, 12B, 12C, 13A, 13B, 13C, 14A and 14B), such as a variety of games, self-assessment, medication alert, and so on. The device integration application allows toggling between the various options on the main menu 612 by going back from one option 612a to the main menu and selecting another option. The variety of games are designed to test speed and accuracy of the user, such as a patient suffering from Parkinson's disease. In an example, one of the variety of games include drawing a spiral and/or a straight line between the two points shown on the screen using a finger. The user 606 can take multiple attempts that helps in improving movement. In another example, one of the variety of games include identifying and tracking a change in the display, for example, popping as many balloons as possible within a time limit using the finger, tapping alternating dots on the screen as fast as possible within a time limit using the finger, and so forth. The self-assessment comprises a questionnaire for the user. The user is required to fill out the self-assessment questionnaire in routine, for example weekly, to track and inform doctor or carer about the user's health. The questionnaire may require the user to choose from a predefined scale of responses ranging for example from "None, Slight, Moderate, Severe and Unable" to answer for questions such as "problems with walking", "problems with washing or dressing"," problems with daily activities", "pain/discomfort", "anxiety/depression", and so forth. The device integration application allows the user 606 to move to a new question after registering an answer for the present question. Moreover, the device integration application allows the user 606 to evaluate an overall health (quality of life (QOL)) on a scale of 0 to 100, where 0 is the worst and 100 is the best health the user 606 can imagine. Furthermore, the device integration application provides Medication Alerts, such as the medication next due, and a time in 12- or 24-hour format, for the medication. Additionally, a log is provided for the user 606 to register an answer (in a Yes or a No) if the said alert was addressed and the medication was taken due for that time. Furthermore, a Settings icon allows the user 606 to set alarms, provide prescription details (i.e. medicines and doses) and the frequency for each medicine. Beneficially, the device integration application enables sending the data recorded by the user 606 to respective physicians for tracking the progress of the user 606.

In some embodiments, the wearable device 100, 200, 300, 500, 600 is used for modulating a sensory dysfunction or relieving a physical symptom associated with a neurological condition or disease. The wearable device 100, 200, 300, 500, 600 comprises at least one stimulating element configured to provide at least one mechanical stimulus to a user, wherein the mechanical stimulus comprises a pulsed output; and a dissipating portion configured to increase an effective area of a mechanical stimulus provided by the at least one stimulating element, the dissipating portion coupled with the at least one stimulating element, wherein the wearable device 100, 200, 300, 500, 600, when in operation, is in physical contact with body of a user. The user may be suffering from a neurological condition or disease, for example Parkinson's disease, Alzheimer's disease, multiple sclerosis. In aforesaid diseases, the patient suffers from a neurological condition that affects their movement. In this regard, the wearable device 100, 200, 300, 500, 600 is configured to enable the at least one stimulating element to generate pulsed output. Consequently, the pulsed output provides effective but gentle electrical (or vibrotactile) stimulations serving as cue signs for the user. The pulsed output comprises a regular, rhythmic or periodic fluctuation that may be generated as a continuous waveform or as binary mechanical stimulus. The binary mechanical stimulus provides a tactile cue sign, that enables modulation sensory dysfunction, and enables users to focus on the cues provided by the pulsed output to coordinate timings of their own movement to the pulsed output. In doing so, freeze of gait, tremors, stiffness or slowness, associated with the aforesaid medical conditions, may be further reduced. Beneficially, the tactile cue sign aids in improving movement and task completion as the user may inherently be aware of the mechanical stimulus or tactile cue sign without requiring active focus while being detracted from concentrating on a desired task.

In some embodiments, the pulsed output is a balanced pulsed output. The balanced pulsed output results in symmetrical positive portion (cathodic pulse) characterized by high amplitude and negative portion (anodic pulse) characterized by very low amplitude, preferably zero, but with substantially equal pulse durations. Moreover, the pulsed output results in 6 to 50% improvement in the user, preferably 32% improvement. The improvement of up to 32% is observed for the activation of neurons as well as coordination of movement based on the pulsed stimulation. Furthermore, the movement coordination is associated with user's focus on the cues provided by the pulsed output to coordinate timings of their own movement to the pulsed output.

In some embodiments, the wearable device 100, 200, 300, 500, 600 makes the physical contact with body of the user by a mechanical engagement, and wherein the mechanical engagement is by any of: an adhesive, a strap, a locket, a bracelet, a band, a belt, a vacuum cup, a magnet. The physical contact with the body of the user, i.e. skin on the user, an effective the transmission of the tactile stimulus from the wearable device 100, 200, 300, 500, 600 to the user. The wearable device 100, 200, 300, 500, 600 may be placed against any body part such as sternum, arm, shoulder, wrist, neck, ankle, leg and temple. The aforementioned body parts allow for easy access of the wearable device 100, 200, 300, 500, 600 by the user and by a carer or family member of an incapacitated user. The appealing design of the wearable device 100, 200, 300, 500, 600 serves the device to be flaunted as an ornament. However, some users may be not be comfortable in showing off the wearable device 100, 200, 300, 500, 600 and may wear the wearable device 100, 200, 300, 500, 600 at any other body part hidden under a piece of cloth for example. For example, the wearable device 100, 200, 300, 500, 600 may be placed against thighs or on the back of the user.

The reservoir for adhesive is provided in the base of the docking station or the lid of the docking station, and wherein the reservoir rotatably removable from the base of the docking station or a lid of the docking station. The wearable device 100, 200, 300, 500, 600 is attachable to the skin of the user, preferably sternum, by for example adhesive. The adhesive is provided as consumable pads of hook-and loop material comprising layers of adhesives on the back side thereof. As discussed above, a medical grade adhesive is provided at the back of the loop material that is placed facing towards the skin of the user, and a stronger adhesive is provided at the back of the hook material that is placed facing towards the wearable device, such as on the dissipating portion thereof. The hook and loop material attach together by way of hooks and loops and hold the wearable device in physical contact with body of the user. The consumable pads of adhesive are supplied and stored in layers in the reservoir for adhesive. The unique design of the reservoir enables accessing the stored consumable pads of adhesive when the consumable pads of adhesive presently in use with the wearable device is unable to show the desired effect. Beneficially, the rotating outwards of the reservoir enables keeping the consumable pads of adhesive in place and enhancing the longevity of the docking station by subjecting it to least number of jerks and orientational changes.

The present disclosure provides a wearable device for providing vibrational stimulation to the users with restricted movement, such as in case of Parkinson's disease. The wearable device of the present disclosure uses a combination of focused stimulation and cueing to generate the vibrational stimulations to help the users regain freedom of movement. The wearable device is sleek and visually appealing, besides extremely comfortable for the users. Moreover, the vibrational stimulation is comfortable to the user, in terms of noise control, heat dissipation, etc. The wearable device is tailored to suit the requirements of the user, such as in terms of altering variables of the stimulation to refine the pattern, strength and frequency from which the user experiences greatest benefits. The wearable device provides improved gait and a better quality of life for people with Parkinson's. The wearable device is a non-invasive treatment for Parkinson's Disease, and the impact of vibrotactile stimulation produced thereby may be extended in the context of rehabilitation programmes and demonstrate long term benefits of this technology. The wearable device can induce changes in the brain related to neuroplasticity and increase responsiveness to the stimulations. Furthermore, the wearable device is a stand-alone device and can be operated by a device integration application software for recording and analysing the overall health (quality of life) of the user.

From reading the present disclosure, other variations and modifications will be apparent to the skilled person. Such variations and modifications may involve equivalent and other features which are already known in the art of wearable devices, and which may be used instead of, or in addition to, features already described herein.

The present invention is defined by the appended claims.

## Claims

1. A wearable device (100) to provide at least one mechanical stimulus to a user with a neurological condition, comprising:
at least one stimulating element (102) configured to provide the at least one mechanical stimulus to the user, wherein an output of the at least one stimulating element (102) comprises a pulsed output;
a controller (320) configured to control an output of the at least one stimulating element (102); and
a dissipating portion (104) configured to increase an effective area of a mechanical stimulus provided by the at least one stimulating element (102);
**characterized in that** the pulsed output provides a combination of focused vibrotactile stimulation and cueing to the user.

2. The wearable device (100) of claim 1, further comprising a surface (306) configured to deliver and/or transmit the mechanical stimulus to the user, optionally at least a part of the dissipating portion (102) is configured to form at least part of the surface (106) configured to deliver the mechanical stimulus to the user.

3. The wearable device (100) of claim 2, wherein the at least one stimulating element (102) is configured to form at least part of the surface (106) configured to deliver a mechanical stimulus to the user.

4. The wearable device (100) of any preceding claim, wherein the dissipating portion (104) comprises a recess or aperture configured to receive the at least one stimulating element (102), optionally the at least one stimulating element (102) is substantially flush with a mouth of the recess or aperture when the at least one stimulating element (102) is received by the recess or aperture.

5. The wearable device (100) of any claim dependent directly or indirectly from claim 2, wherein the surface (106) configured to deliver a mechanical stimulus to the user comprises an adhesive configured to adhere the surface (106) to skin of the user.

6. The wearable device (100) of any preceding claim, further comprising an electric charging portion (216), optionally the electric charging portion (216) is configured to receive electric power wirelessly from an external electric power source and/or the electric charging portion (216) is configured to receive electric power from an external electric power source by a wired connection.

7. The wearable device (100) of any preceding claim, wherein the pulsed output is or comprises a periodic output, and optionally wherein a frequency of the periodic output is between substantially 4 Hz and 0.25 Hz, and further optionally wherein a frequency of the periodic output is substantially 1 Hz.

8. The wearable device (100) of claim 7, wherein each period of the pulsed output comprises at least a first segment and a second segment and, optionally, where the first segment is different to the second segment in one or more of output intensity, frequency and duration.

9. The wearable device (100) of claim 8, wherein:
the first segment comprises increasing an output intensity of the at least one stimulating element (102) from a first intensity level to a second intensity level; and
the second segment comprises decreasing an output intensity of the at least one stimulating element (102) from the second intensity level to the first intensity level.

10. The wearable device (100) of claim 9, wherein a rate of increase in output intensity in the first segment is different from a rate of decrease in output intensity in the second segment; and, optionally, wherein the rate of increase in output intensity in the first segment is lower than the rate of decrease in output intensity in the second segment; and, further optionally, wherein the rate of increase in output intensity in the first segment is between substantially three and ten times lower than the rate of decrease in output intensity in the second segment.

11. The wearable device (100) of any preceding claim, wherein a user input is configured to receive at least:
a first command to cause the controller to instruct the at least one stimulating element (102) to provide a first output, and
a second command, different from the first command, to cause the controller to instruct the at least one stimulating element (102) to provide a second output.

12. The wearable device (100) of any preceding claim, wherein the vibrotactile stimulation is a periodic signal having a frequency between substantially 300 Hz and 0 Hz.

13. The wearable device (100) of any preceding claim, wherein the at least one stimulating element (102) comprises a motor configured to provide a vibrotactile stimulus to the user, optionally wherein the output speed of the motor is between substantially 5000 RPM and 20000 RPM.

14. A kit of parts comprising:
a wearable device (100) of any of the preceding claims; and
a docking station (250) for receiving and storing the wearable device (100), wherein the docking station (250) is a single unit comprising a base (274) and a lid (272) removably attached at an end.

## Patentansprüche

1. Tragbare Vorrichtung (100) zum Bereitstellen zumindest eines mechanischen Stimulus für einen Benutzer mit einer neurologischen Erkrankung, umfassend:
zumindest ein Stimulationselement (102), das konfiguriert ist, um dem Benutzer den zumindest einen mechanischen Stimulus bereitzustellen, wobei eine Ausgabe des zumindest einen Stimulationselements (102) eine gepulste Ausgabe umfasst;
eine Steuerung (320), die konfiguriert ist zum Steuern einer Ausgabe des zumindest einen Stimulationselements (102); und
einen Streubereich (104), der konfiguriert ist, um eine Wirkungsfläche eines mechanischen Stimulus zu erhöhen, der von dem zumindest einen Stimulationselement (102) bereitgestellt wird;
**dadurch gekennzeichnet, dass** die gepulste Ausgabe eine Kombination aus einer gezielten vibrotaktilen Stimulation und Hinweisen für den Benutzer bereitstellt.

2. Tragbare Vorrichtung (100) nach Anspruch 1, ferner umfassend eine Oberfläche (306), die konfiguriert ist, um den mechanischen Stimulus an den Benutzer abzugeben und/oder zu übertragen, wobei wahlweise zumindest ein Teil des Streubereichs (102) konfiguriert ist, um zumindest einen Teil der Oberfläche (106) zu bilden, die konfiguriert ist, um den mechanischen Stimulus an den Benutzer abzugeben.

3. Tragbare Vorrichtung (100) nach Anspruch 2, wobei das zumindest eine Stimulationselement (102) konfiguriert ist, um zumindest einen Teil der Oberfläche (106) zu bilden, die konfiguriert ist, um einen mechanischen Stimulus an den Benutzer abzugeben.

4. Tragbare Vorrichtung (100) nach einem der vorhergehenden Ansprüche, wobei der Streubereich (104) eine Aussparung oder Öffnung umfasst, die konfiguriert ist, um das zumindest eine Stimulationselement (102) aufzunehmen, wobei optional das zumindest eine Stimulationselement (102) im Wesentlichen mit einer Mündung der Aussparung oder Öffnung fluchtrecht ist, wenn das zumindest eine Stimulationselement (102) von der Aussparung oder Öffnung aufgenommen wird.

5. Tragbare Vorrichtung (100) nach einem beliebigen Anspruch in direkter oder indirekter Abhängigkeit von Anspruch 2, wobei die Oberfläche (106) die konfiguriert ist, um einen mechanischen Stimulus an den Benutzer abzugeben, einen Klebstoff umfasst, der konfiguriert ist, um die Oberfläche (106) auf die Haut des Benutzers zu kleben.

6. Tragbare Vorrichtung (100) nach einem der vorhergehenden Ansprüche, ferner umfassend einen elektrischen Ladeabschnitt (216), wobei der elektrische Ladeabschnitt (216) optional konfiguriert ist, um elektrische Leistung drahtlos von einer externen elektrischen Stromquelle zu empfangen und/oder der elektrische Ladeabschnitt (216) konfiguriert ist, um elektrische Leistung von einer externen elektrischen Stromquelle über eine Kabelverbindung zu empfangen.

7. Tragbare Vorrichtung (100) nach einem der vorhergehenden Ansprüche, wobei die gepulste Ausgabe eine periodische Ausgabe ist oder umfasst, und wobei optional eine Frequenz der periodischen Ausgabe zwischen im Wesentlichen 4 Hz und 0,25 Hz liegt, und ferner optional eine Frequenz der periodischen Ausgabe im Wesentlichen 1 Hz beträgt.

8. Tragbare Vorrichtung (100) nach Anspruch 7, wobei jede Periode der gepulsten Ausgabe zumindest ein erstes Segment und ein zweites Segment umfasst, und optional, wobei sich das erste Segment von dem zweiten Segment durch eine oder mehrere der Ausgabeintensität, Frequenz und Dauer unterscheidet.

9. Tragbare Vorrichtung (100) nach Anspruch 8, wobei:
das erste Segment das Erhöhen einer Ausgabeintensität des zumindest einen Stimulationselements (102) von einem ersten Intensitätsniveau auf ein zweites Intensitätsniveau umfasst; und
das zweite Segment das Verringern einer Ausgabeintensität des zumindest einen Stimulationselements (102) von dem zweiten Intensitätsniveau auf das erste Intensitätsniveau umfasst.

10. Tragbare Vorrichtung (100) nach Anspruch 9, wobei eine Erhöhungsrate der Ausgabeintensität in dem ersten Segment sich von einer Verringerungsrate der Ausgabeintensität in dem zweiten Segment unterscheidet; und wobei optional die Erhöhungsrate der Ausgabeintensität in dem ersten Segment niedriger als die Verringerungsrate der Ausgabeintensität in dem zweiten Segment ist; und wobei optional ferner die Erhöhungsrate der Ausgabeintensität in dem ersten Segment zwischen im Wesentlichen drei- und zehnmal niedriger als die Verringerungsrate der Ausgabeintensität in dem zweiten Segment ist.

11. Tragbare Vorrichtung (100) nach einem der vorhergehenden Ansprüche, wobei eine Benutzereingabe konfiguriert ist, um zumindest Folgendes zu empfangen:
einen ersten Befehl, um die Steuerung zu veranlassen, das zumindest eine Stimulationselement (102) anzuweisen, eine erste Ausgabe bereitzustellen, und
einen zweiten Befehl, der sich von dem ersten Befehl unterscheidet, um die Steuerung zu veranlassen, das zumindest eine Stimulationselement (102) anzuweisen, eine zweite Ausgabe bereitzustellen.

12. Tragbare Vorrichtung (100) nach einem der vorhergehenden Ansprüche, wobei die vibrotaktile Stimulation ein periodisches Signal ist, das im Wesentlichen eine Frequenz zwischen 300 Hz und 0 Hz aufweist.

13. Tragbare Vorrichtung (100) nach einem der vorhergehenden Ansprüche, wobei das zumindest eine Stimulationselement (102) einen Motor umfasst, der konfiguriert ist, um einen vibrotaktilen Stimulus für den Benutzer bereitzustellen, wobei die Ausgabegeschwindigkeit des Motors optional zwischen im Wesentlichen 5000 U/min und 20000 U/min liegt.

14. Teilesatz, umfassend:
eine tragbare Vorrichtung (100) nach einem der vorhergehenden Ansprüche; und
eine Docking-Station (250) zum Empfangen und Lagern der tragbaren Vorrichtung (100), wobei die Docking-Station (250) eine einzelne Einheit ist, umfassend eine Basis (274) und einen Deckel (272), der an einem Ende abnehmbar angebracht ist.

## Revendications

1. Dispositif portable (100) pour fournir au moins un stimulus mécanique à un utilisateur présentant une affection neurologique, comprenant :
au moins un élément de stimulation (102) configuré pour fournir l'au moins un stimulus mécanique à l'utilisateur, dans lequel une sortie de l'au moins un élément de stimulation (102) comprend une sortie à impulsions ;
un dispositif de commande (320) configuré pour commander une sortie de l'au moins un élément de stimulation (102) ; et
une portion de dissipation (104) configurée pour augmenter une zone efficace d'un stimulus mécanique fourni par l'au moins un élément de stimulation (102) ;
**caractérisé en ce que** la sortie à impulsions fournit une combinaison de stimulation vibrotactile focalisée et de repérage à l'utilisateur.

2. Dispositif portable (100) selon la revendication 1, comprenant en outre une surface (306) configurée pour distribuer et/ou transmettre le stimulus mécanique à l'utilisateur, éventuellement au moins une partie de la portion de dissipation (102) est configurée pour former au moins une partie de la surface (106) configurée pour distribuer le stimulus mécanique à l'utilisateur.

3. Dispositif portable (100) selon la revendication 2, dans lequel l'au moins un élément de stimulation (102) est configuré pour former au moins une partie de la surface (106) configurée pour distribuer un stimulus mécanique à l'utilisateur.

4. Dispositif portable (100) selon l'une quelconque des revendications précédentes, dans lequel la portion de dissipation (104) comprend un évidement ou un orifice configuré pour recevoir l'au moins un élément de stimulation (102), éventuellement l'au moins un élément de stimulation (102) est sensiblement de niveau avec une embouchure de l'évidement ou de l'orifice lorsque l'au moins un élément de stimulation (102) est reçu par l'évidement ou l'orifice.

5. Dispositif portable (100) selon l'une quelconque des revendications dépendant directement ou indirectement de la revendication 2, dans lequel la surface (106) configurée pour distribuer un stimulus mécanique à l'utilisateur comprend un adhésif configuré pour faire adhérer la surface (106) à la peau de l'utilisateur.

6. Dispositif portable (100) selon l'une quelconque des revendications précédentes, comprenant en outre une portion de charge électrique (216), éventuellement la portion de charge électrique (216) est configurée pour recevoir de la puissance électrique sans fil à partir d'une source de puissance électrique externe et/ou la portion de charge électrique (216) est configurée pour recevoir de la puissance électrique à partir d'une source de puissance électrique externe par une connexion câblée.

7. Dispositif portable (100) selon l'une quelconque des revendications précédentes, dans lequel la sortie à impulsions est ou comprend une sortie périodique, et éventuellement dans lequel une fréquence de la sortie périodique est entre sensiblement 4 Hz et 0,25 Hz, et éventuellement en outre dans lequel une fréquence de la sortie périodique est de sensiblement 1 Hz.

8. Dispositif portable (100) selon la revendication 7, dans lequel chaque période de la sortie à impulsions comprend au moins un premier segment et un deuxième segment et, éventuellement, dans lequel le premier segment est différent du deuxième segment dans un ou plusieurs éléments parmi l'intensité, la fréquence et la durée de sortie.

9. Dispositif portable (100) selon la revendication 8, dans lequel :
le premier segment comprend l'augmentation d'une intensité de sortie de l'au moins un élément de stimulation (102) d'un premier niveau d'intensité à un deuxième niveau d'intensité ; et
le deuxième segment comprend la diminution d'une intensité de sortie de l'au moins un élément de stimulation (102) du deuxième niveau d'intensité au premier niveau d'intensité.

10. Dispositif portable (100) selon la revendication 9, dans lequel une vitesse d'augmentation de l'intensité de sortie dans le premier segment est différente d'une vitesse de diminution de l'intensité de sortie dans le deuxième segment ; et, éventuellement, dans lequel la vitesse d'augmentation de l'intensité de sortie dans le premier segment est inférieure à la vitesse de diminution de l'intensité de sortie dans le deuxième segment ; et, en outre éventuellement, dans lequel la vitesse d'augmentation de l'intensité de sortie dans le premier segment est entre sensiblement trois et dix fois inférieure à la vitesse de diminution de l'intensité de sortie dans le deuxième segment.

11. Dispositif portable (100) selon l'une quelconque des revendications précédentes, dans lequel une entrée d'utilisateur est configurée pour recevoir au moins :
un premier ordre pour amener le dispositif de commande à donner l'instruction à l'au moins un élément de stimulation (102) de fournir une première sortie, et
un deuxième ordre, différent du premier ordre, pour amener le dispositif de commande à donner l'instruction à l'au moins un élément de stimulation (102) de fournir une deuxième sortie.

12. Dispositif portable (100) selon l'une quelconque des revendications précédentes, dans lequel la stimulation vibrotactile est un signal périodique ayant une fréquence entre sensiblement 300 Hz et 0 Hz.

13. Dispositif portable (100) selon l'une quelconque des revendications précédentes, dans lequel l'au moins un élément de stimulation (102) comprend un moteur configuré pour fournir un stimulus vibrotactile à l'utilisateur, éventuellement dans lequel la vitesse de sortie du moteur est entre sensiblement 5000 tr/min et 20 000 tr/min.

14. Kit de pièces comprenant :
un dispositif portable (100) de l'une quelconque des revendications précédentes ; et
une station d'accueil (250) destinée à recevoir et stocker le dispositif portable (100), dans lequel la station d'accueil (250) est une unité unique comprenant une base (274) et un couvercle (272) fixés de manière amovible au niveau d'une extrémité.
